# EUROPEAN PATENT APPLICATION

(11) **EP 4 458 963 A1**
(43) Date of publication of application: **06.11.2024**
(21) Application number: 23171650.7
(22) Date of filing: 04.05.2023
(51) Int. Cl.: C12N 9/22, C12N 9/78, C12N 15/10, C12N 15/90

(54) **HIGHLY ACTIVE CRISPR BASE EDITORS OBTAINED THROUGH CAS-ASSISTED SUBSTRATE-LINKED DIRECTED EVOLUTION (CASLIDE)**

(71) Applicant: Technische Universität Dresden, 01069 Dresden (DE)
(72) Inventor: SÜRÜN, Duran, 01307 Dresden (DE); BUCHHOLZ, Frank, 01307 Dresden (DE)
(74) Representative: Maikowski & Ninnemann Patentanwälte Partnerschaft mbB

(57) **Abstract**

The invention relates to a DNA modifying enzyme comprising or consisting of a DNA editing protein and a Cas protein and a peptide linker,
wherein said DNA editing protein and said Cas protein are connected by said peptide linker; and
wherein said DNA modifying enzyme has been obtained by directed molecular evolution of an original enzyme; and
wherein said DNA modifying enzyme shows a base editing rate which is at least 10%, 20% or 30 %, preferably at least 40 %, more preferably at least 50 %, most preferably at least 60 % higher than the base editing rate of said wildtype enzyme.

The invention relates further to a method of directed molecular evolution of a DNA modifying enzyme and to the use evolved DNA modifying enzymes in research, medicine and agriculture.

## Description

### Field of the invention

The invention relates to a DNA modifying enzyme comprising or consisting of a DNA editing protein and a Cas protein and a peptide linker,
wherein said DNA editing protein and said Cas protein are connected by said peptide linker; and
wherein said DNA modifying enzyme has been obtained by directed molecular evolution of an original enzyme; and
wherein said DNA modifying enzyme shows a base editing rate which is at least 10%, 20% or 30 %, preferably at least 40 %, more preferably at least 50 %, most preferably at least 60 % higher than the base editing rate of said wildtype enzyme.

The invention relates further to a method of directed molecular evolution of a DNA modifying enzyme and to the use evolved DNA modifying enzymes in research, medicine and agriculture.

### Background of the invention

DNA modifying enzymes, such as CRISPR base editors hold tremendous potential to revolutionize therapies for genetic diseases. Unfortunately, the most efficient base editors (e.g. spCas9- CBE or ABE) are very large and do not fit into prominent viral vectors (e.g. AAV). Recently, miniature CRISPR systems have been identified (e.g. Cas12f), but these enzymes are not active enough to allow efficient base editing in mammalian cells.

Directed evolution applies the principles of Darwinian evolution to the laboratory to improve protein features (Li, H., 2021; Danecek, P. et al., 2021). During rounds of mutagenesis and selection, large gene variant libraries (~10⁵ - ~10⁸) are produced (Lawrence, M. et al., 2013; Rognes, T. et al., 2016; Vaser R. et al., 2017). Screening libraries to identify efficient variants is conventionally a manual process that is labor, resource, and time intensive. Furthermore, the number of variants that can be tested is limited, reducing the probability of identifying optimal variants. A high-throughput method for comparing large sets of enzymes would be desirable. While a number of applications have been developed for high-throughput screening of enzyme variants (Tange, O., 2018; Wickham, H. et al., 2019; van der loo, M.P.J., 2014), to our knowledge none of these enable the efficient linkage of genotype and phenotype at scale.

Accordingly, there is a great need in DNA modifying enzymes, such as miniature CRISPR systems with improved DNA editing efficiency and in reliable methods to produce large gene variant libraries of such DNA modifying enzymes, and to screen these large libraries for variants with optimal activity.

### Summary of the invention

Accordingly, it is the problem the invention to provide DNA modifying enzymes with improved base editing efficiency and to provide reliable methods to produce large gene variant libraries of such DNA modifying enzymes.

This problem is solved by the invention by the provision of DNA modifying enzymes of claim 1 and a method of directed molecular evolution of a DNA modifying enzyme according to claim 16.

In particular, the invention provides in first aspect a DNA modifying enzyme comprising or consisting of a DNA editing protein and a Cas protein and a peptide linker,
wherein said base editing protein and said Cas protein are connected by said peptide linker; and
wherein said DNA modifying enzyme has been obtained by directed molecular evolution of an original enzyme; and
wherein said DNA modifying enzyme shows a editing rate which is at least 30 %, preferably at least 40 %, more preferably at least 50 %, most preferably at least 60 % higher than the base editing rate of said original enzyme.

In a second aspect, the invention provides a method of directed molecular evolution of a DNA modifying enzyme as claimed in any one of claims 1 to 15 or of any of the accompanying factors (or components) such as sgRNA scaffold, or pegRNA scaffold in the case of prime editing, said method comprising the steps of
a. Generation of a library of said DNA modifying enzyme,
b. Cloning said library in an expression vector containing the nucleic acid of at least one target site, preferably the nucleic acids of 2 or 3 target sites of said DNA modifying enzyme,
c. Transformation of the expression vector of step b. into a host cell and expression of the library of said DNA modifying enzyme in said host cell;
d. Isolation of plasmids and digestion with the restriction enzymes (RE), which target the nucleic acids of the target sites;
e. Selection of linearized plasmids containing a DNA edition in the nucleic acid of at least one target site, preferably 2 or 3 target sites,
f. Subjecting the linearized plasmids to a PCR or DNA shuffling and repeating steps b. to e. to enrich the library of DNA modifying enzymes.

In further aspects, the invention provides compositions comprising the DNA modifying enzymes of the invention and the use said DNA modifying enzymes or the compositions comprising the same in research, medicine and agriculture.

### Description of the drawings

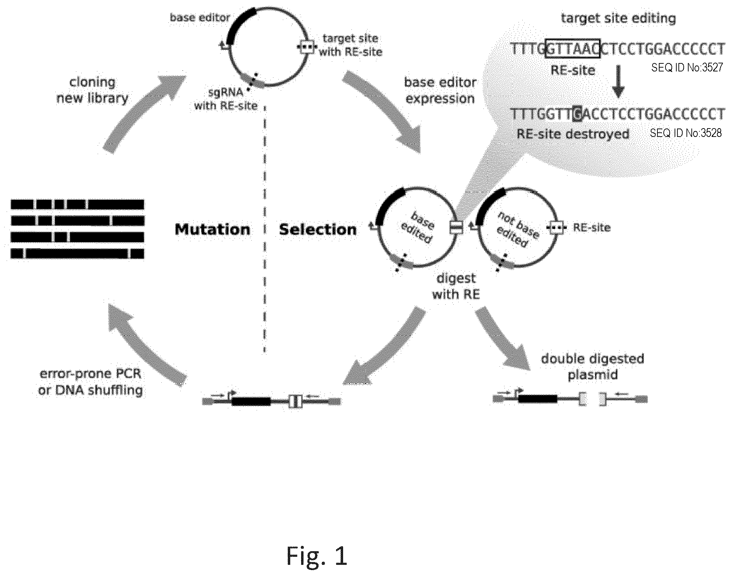

- **Fig. 1**: schematically shows the CRISPR associated substrate linked directed evolution (CaSLiDE) method used in Examples 4 and 9. The gene of a DNA modifying enzyme, which performs base editing is amplified using error-prone PCR or DNA shuffling. This results in multiple copies of the gene with mutations. This gene library is then cloned into a bacterial expression vector, that contains target sites for the base editor, with restriction enzyme (RE) sites located at the position where base change is supposed to happen. The expression vector is transformed into bacteria, which express the DNA modifying enzyme. A sgRNA translated from the same vector guides the base editing enzyme to the target site and causes editing, dependent on the activity of the enzyme. An example for editing can be seen in the bubble in the top right of the figure. Here an A is modified to a G which causes the loss of a RE-site. A digest with the respective RE results in two possible outcomes, the plasmid is digested twice or once. Only single digested plasmids are valid templates for the error-prone PCR (performed with the primers that are indicated as arrows) to start a new cycle of evolution.

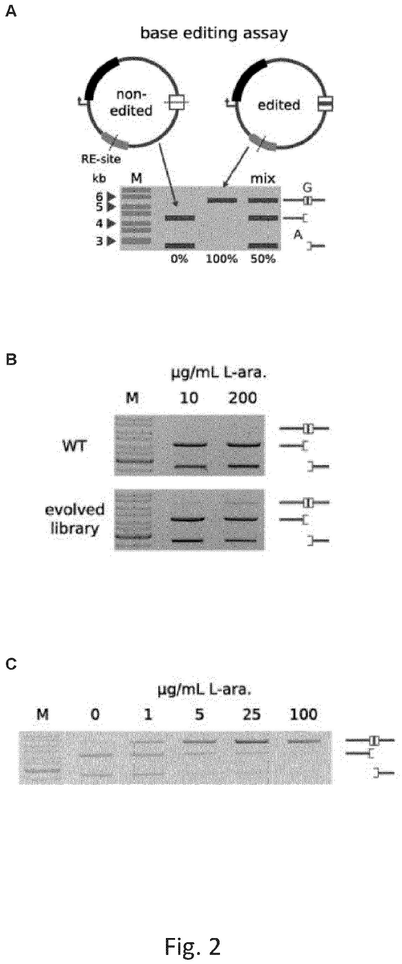

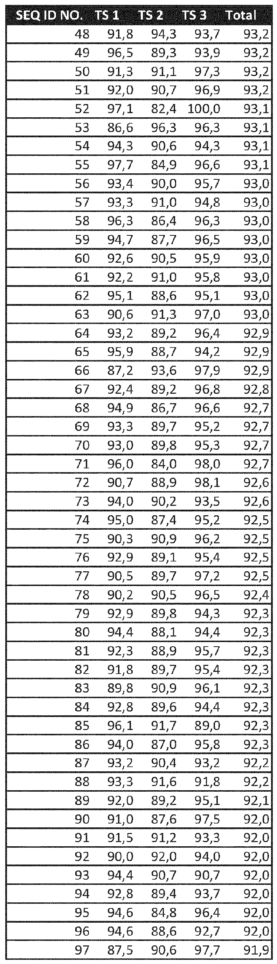

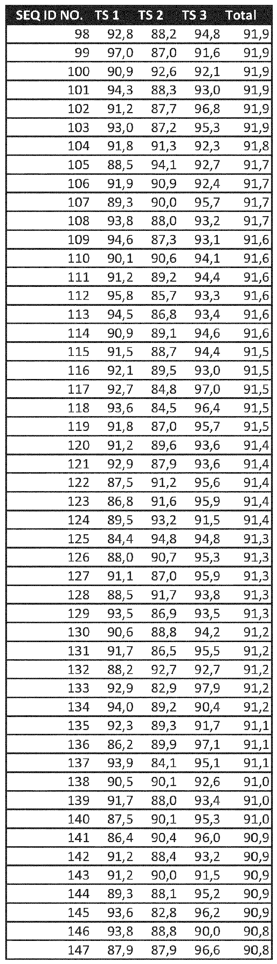

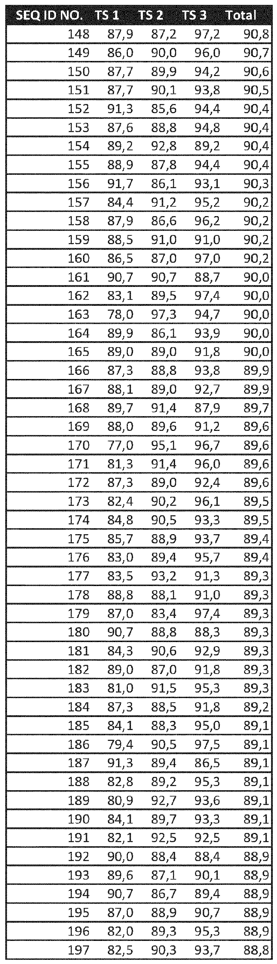

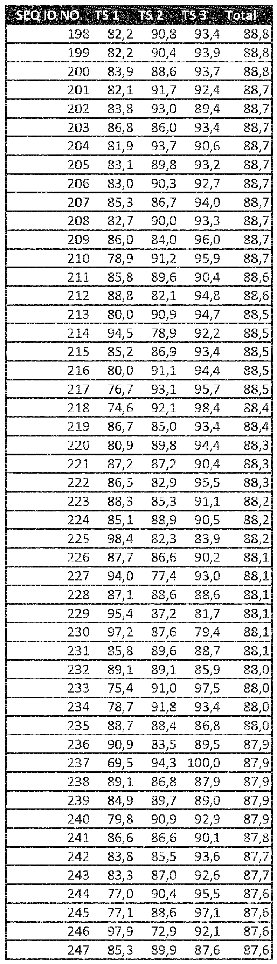

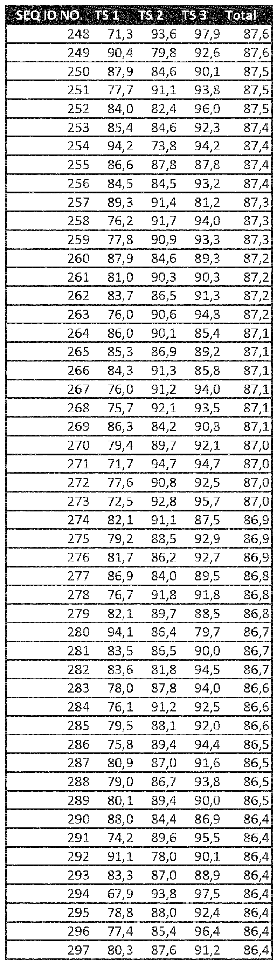

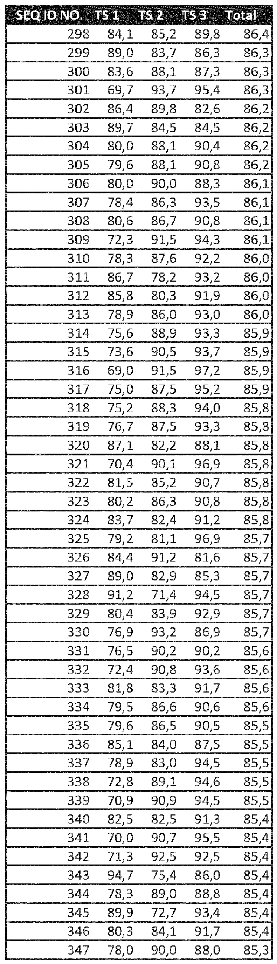

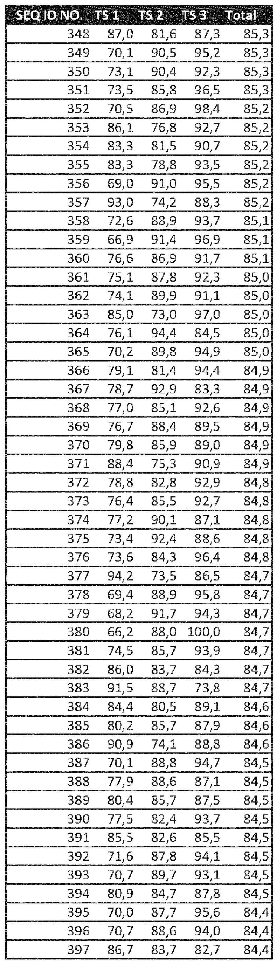

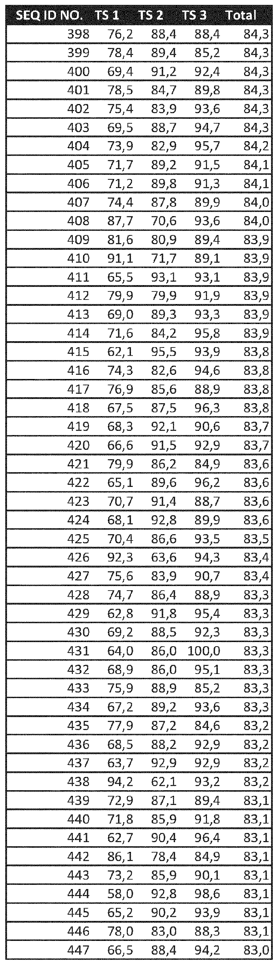

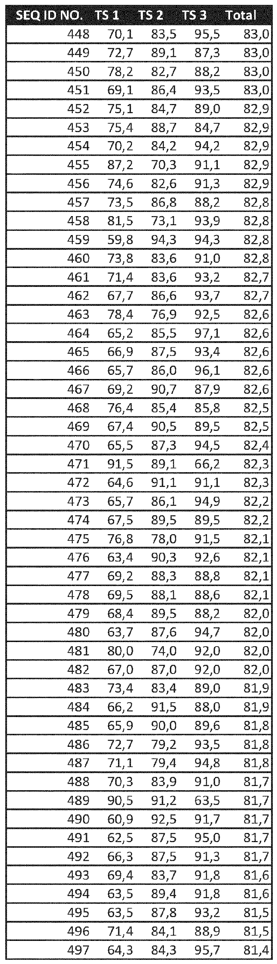

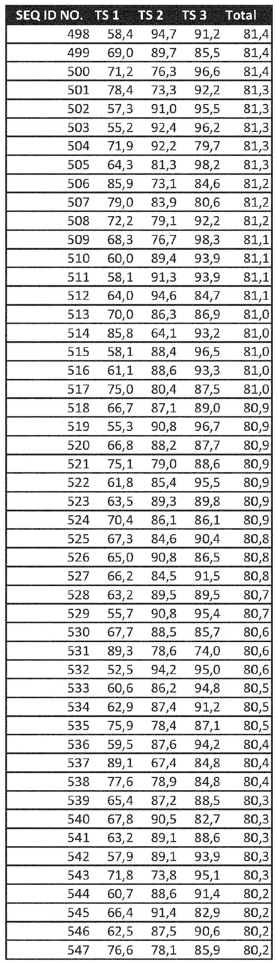

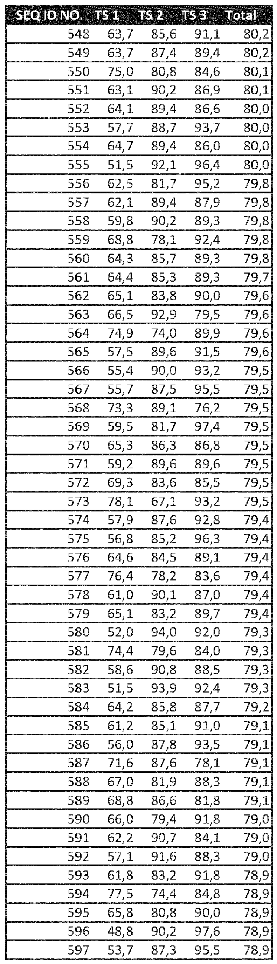

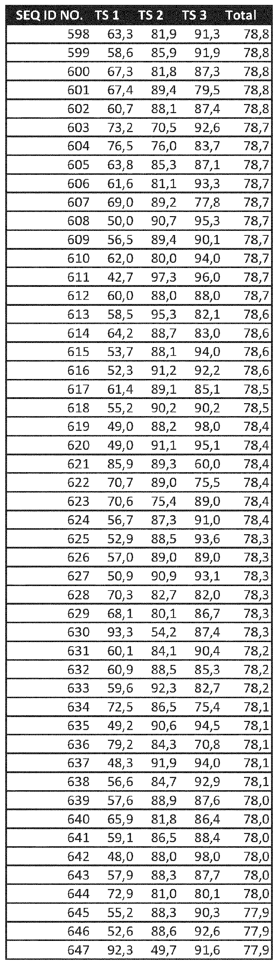

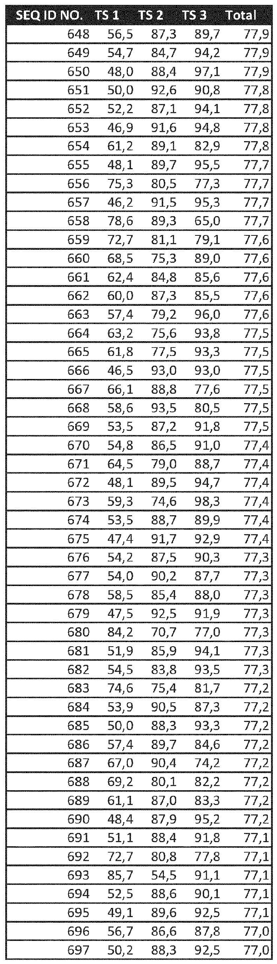

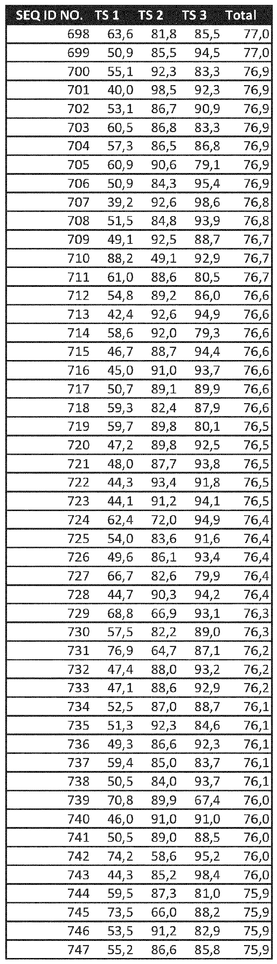

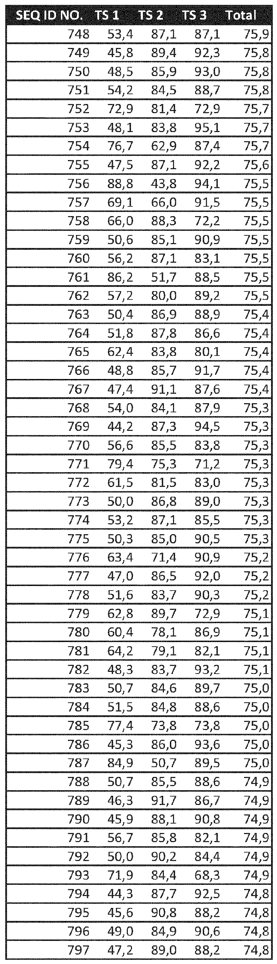

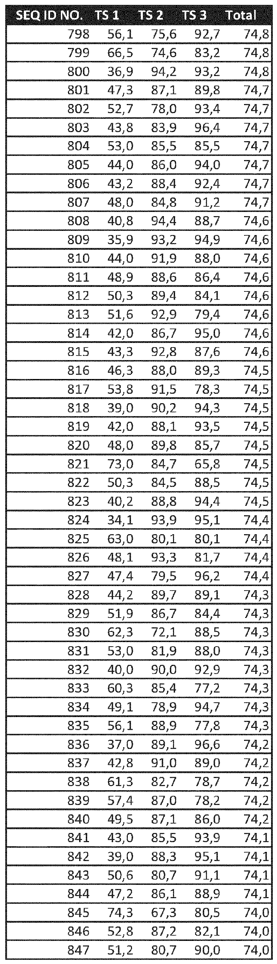

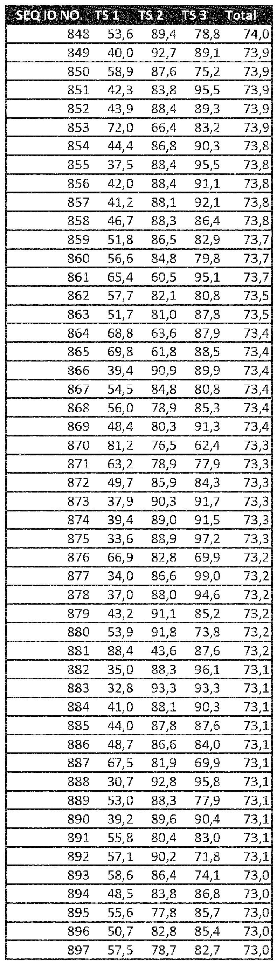

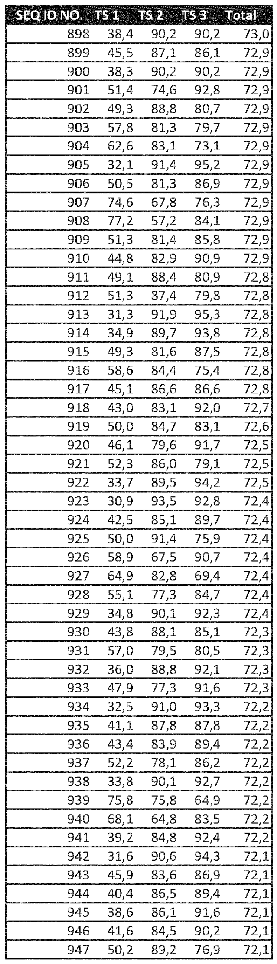

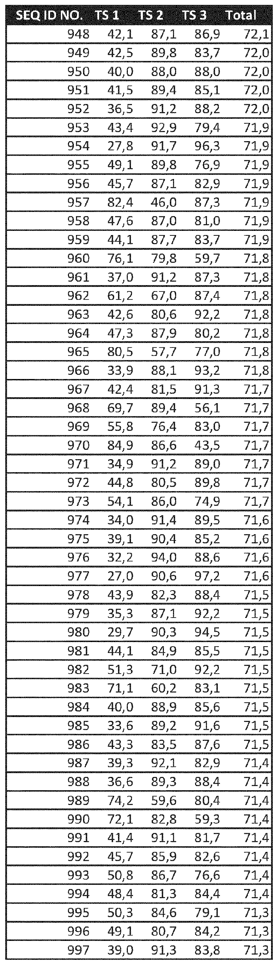

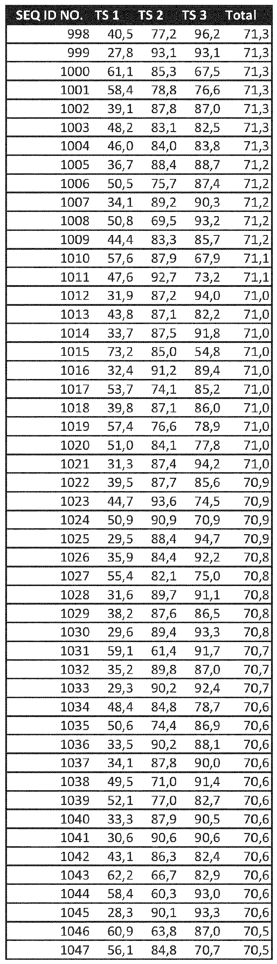

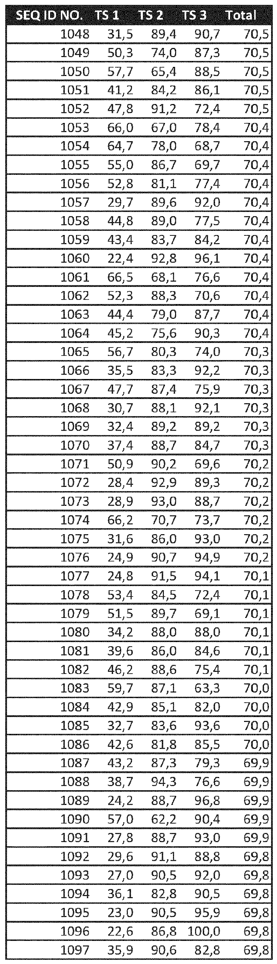

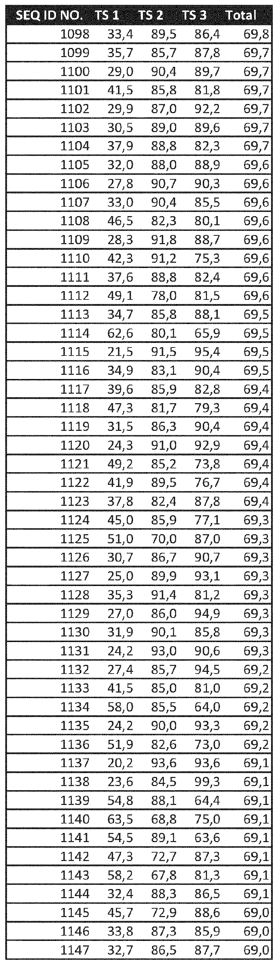

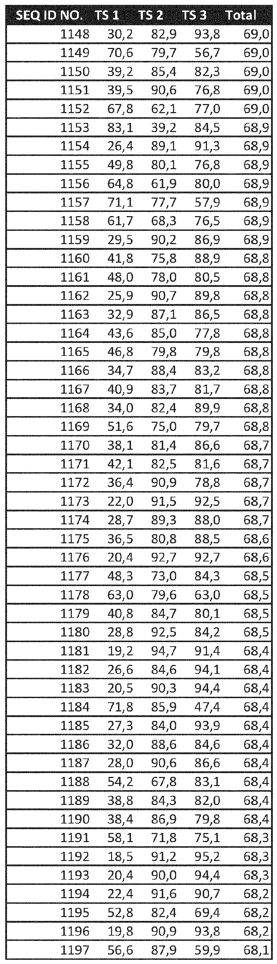

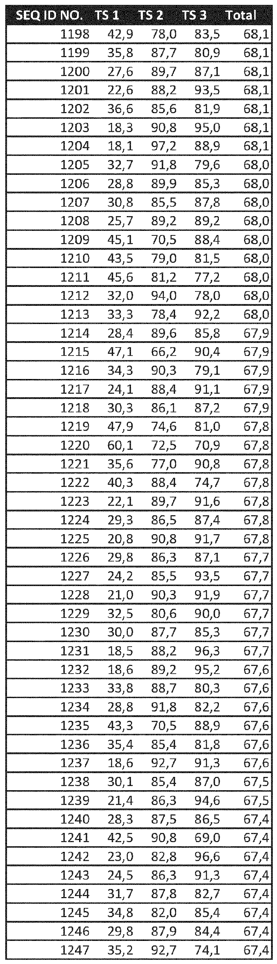

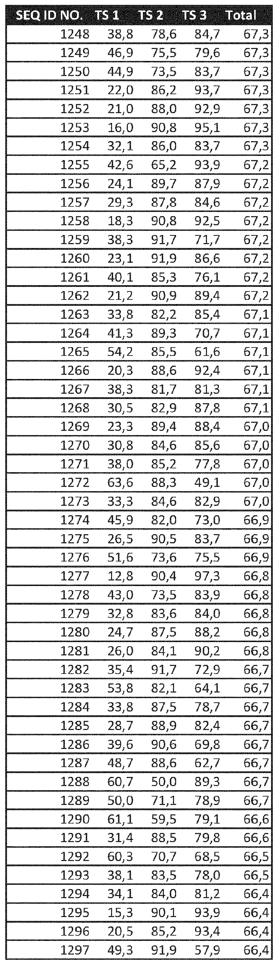

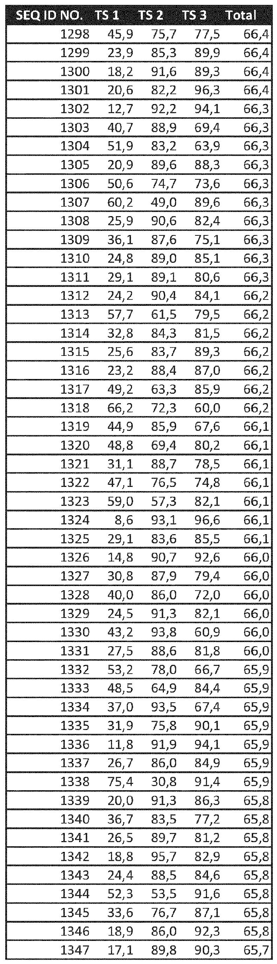

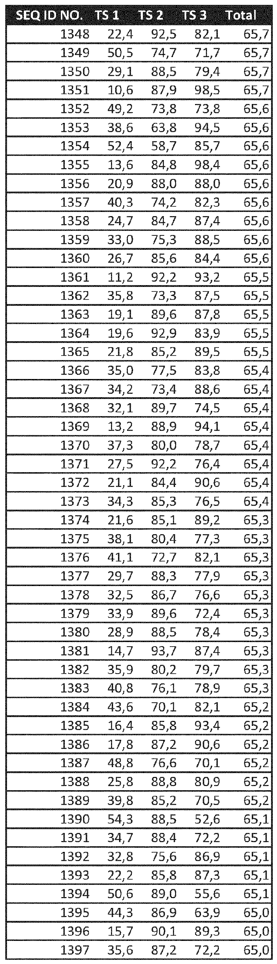

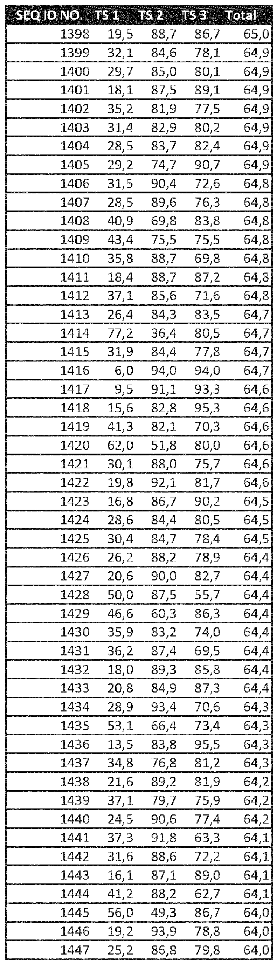

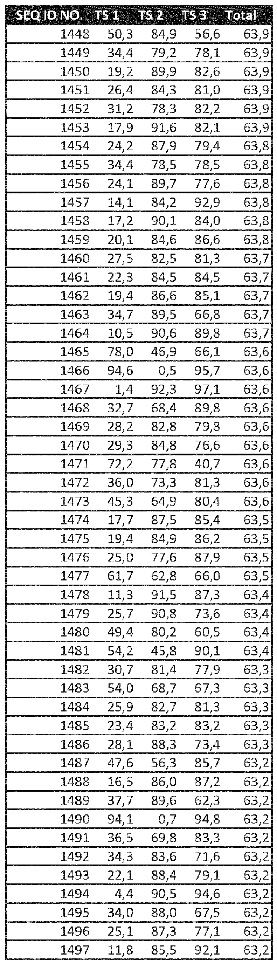

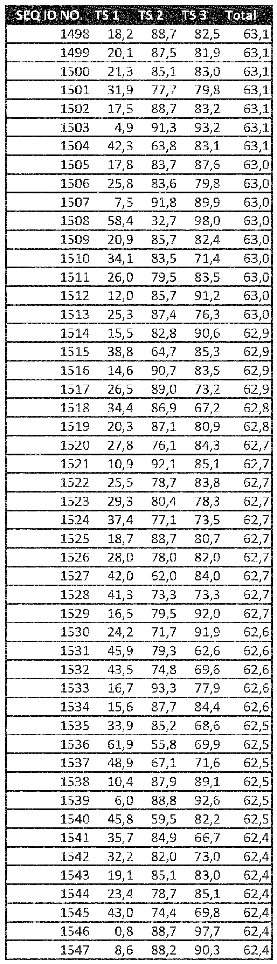

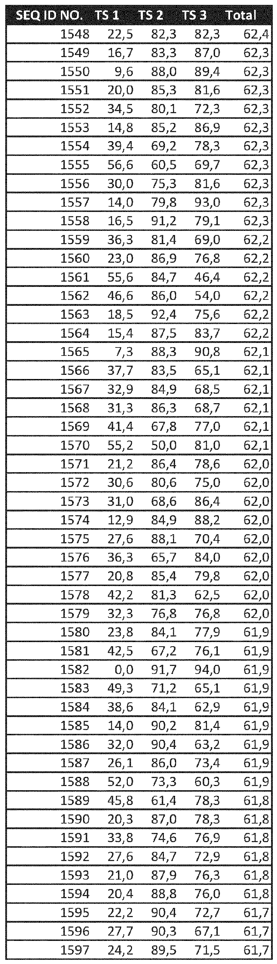

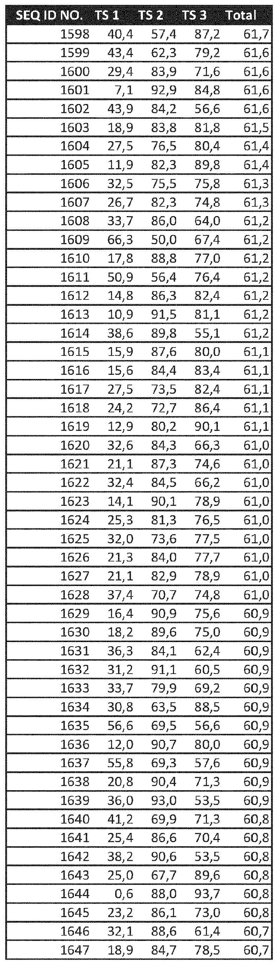

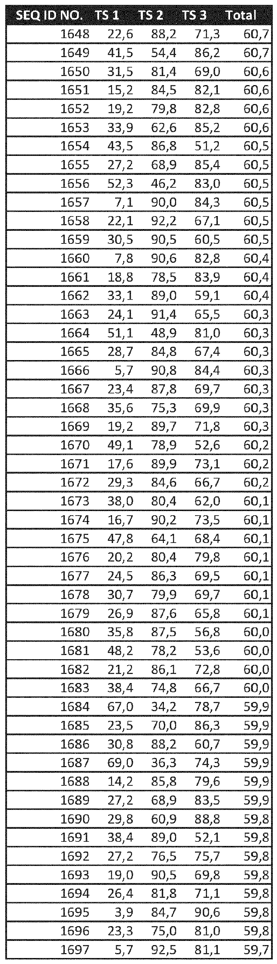

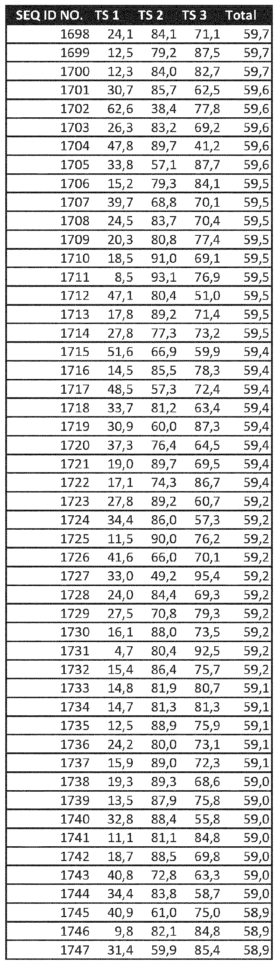

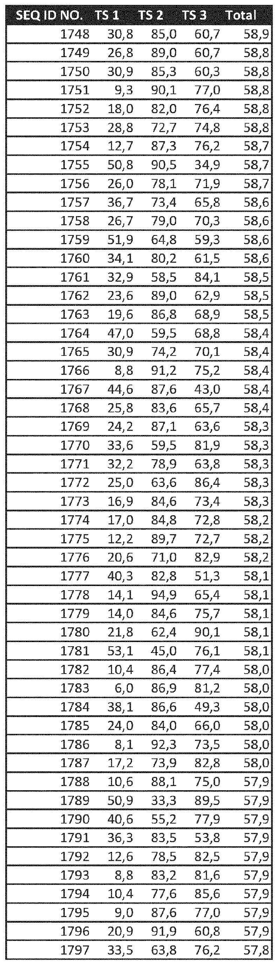

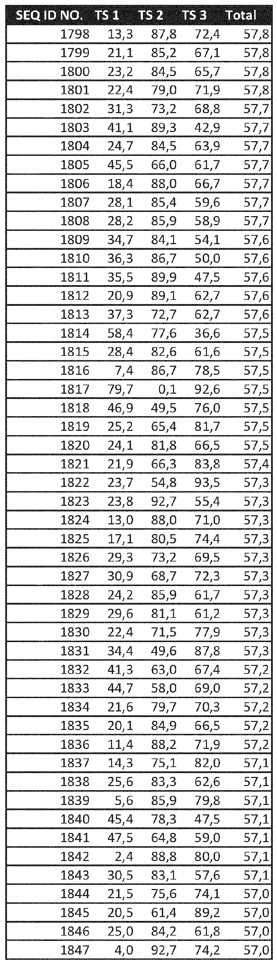

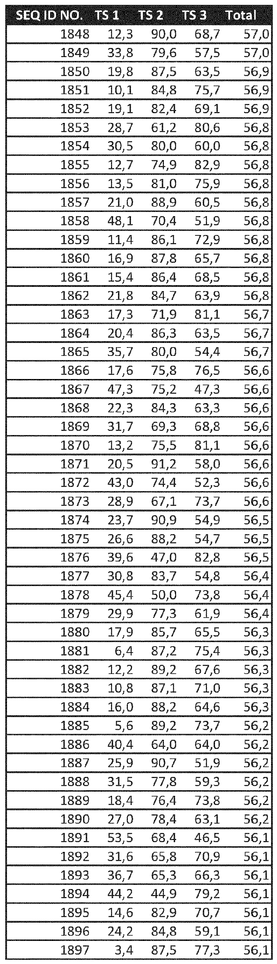

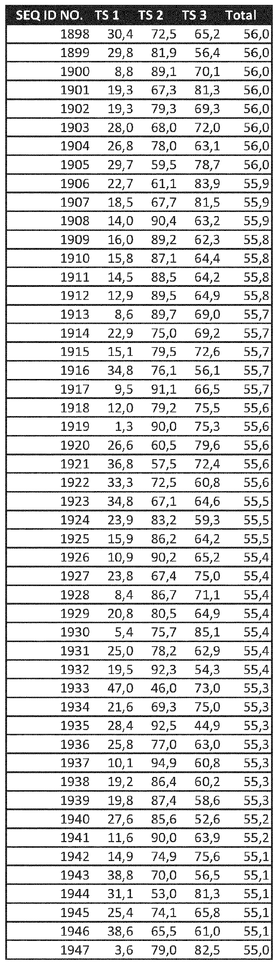

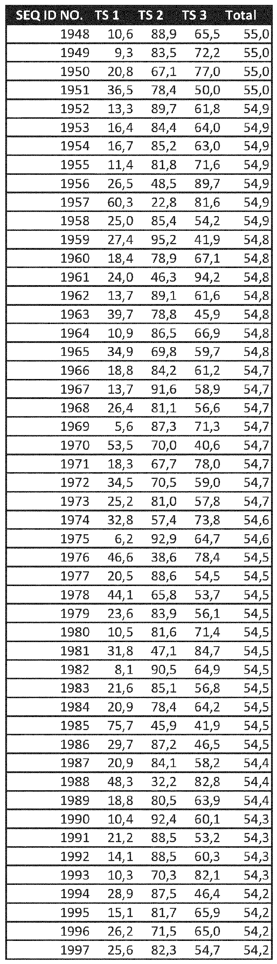

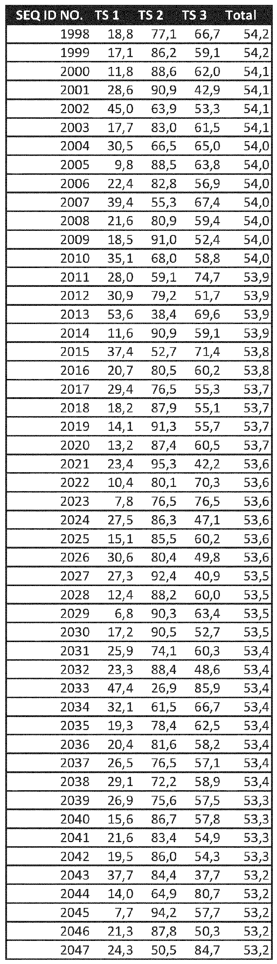

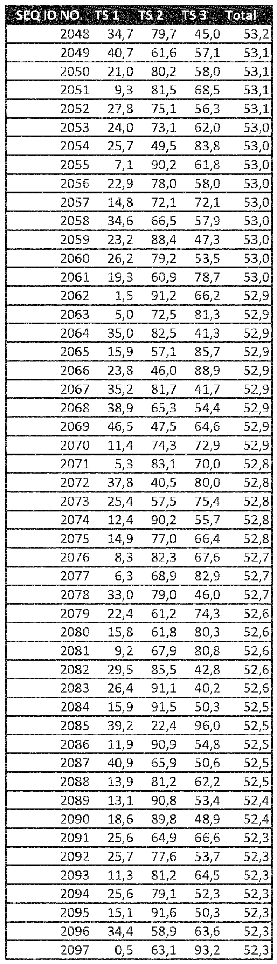

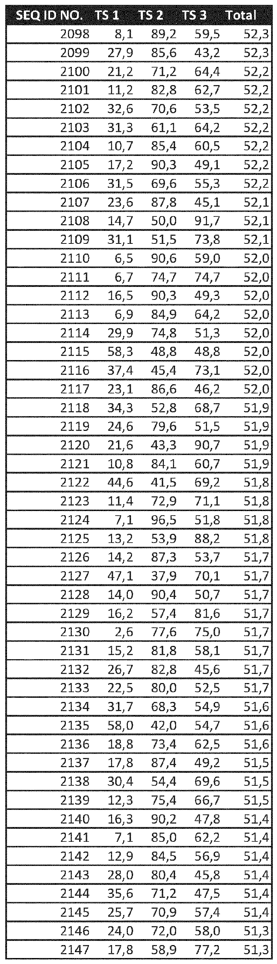

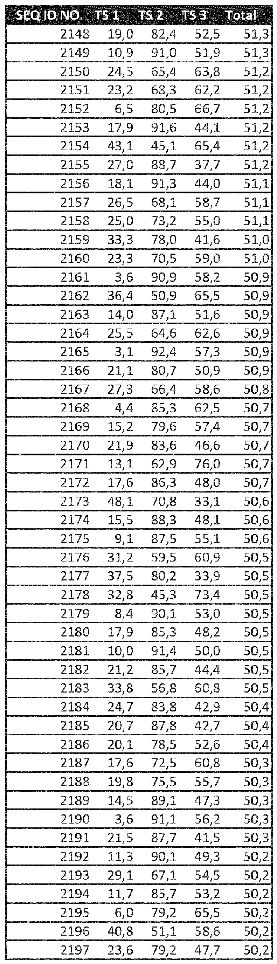

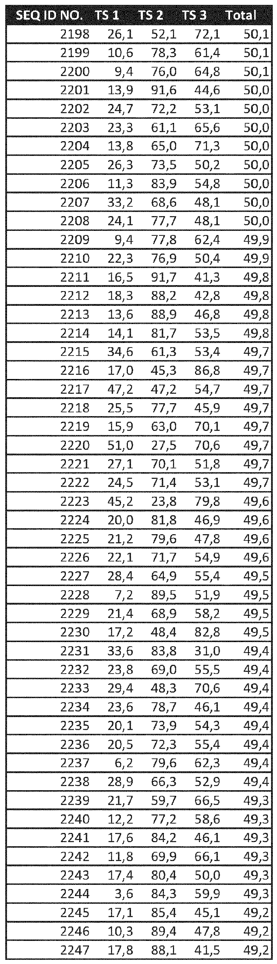

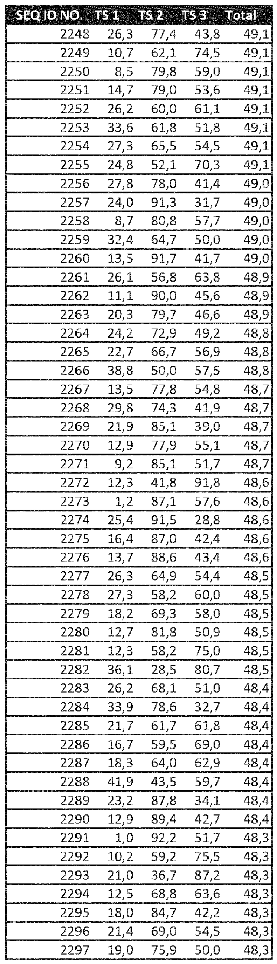

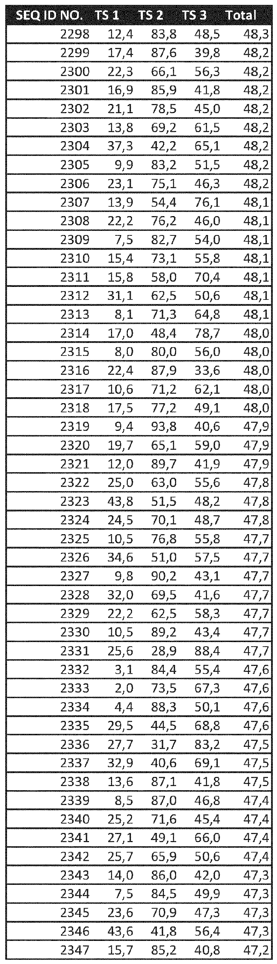

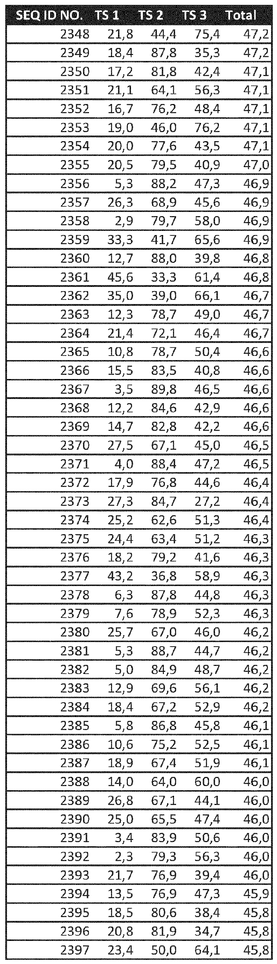

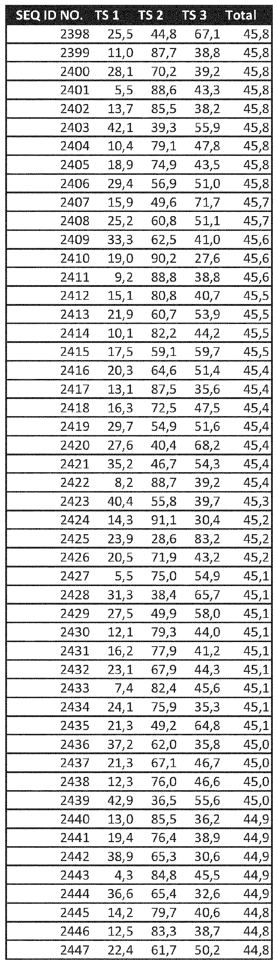

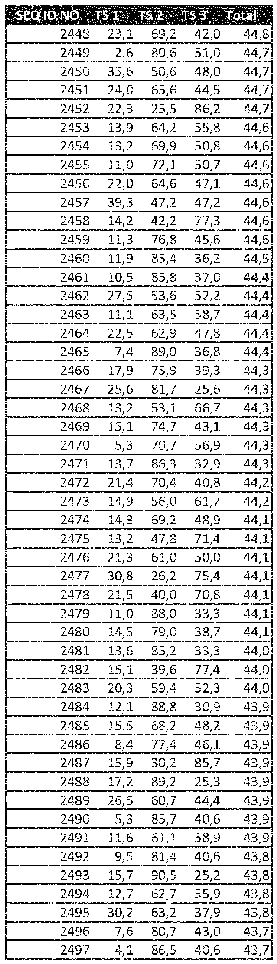

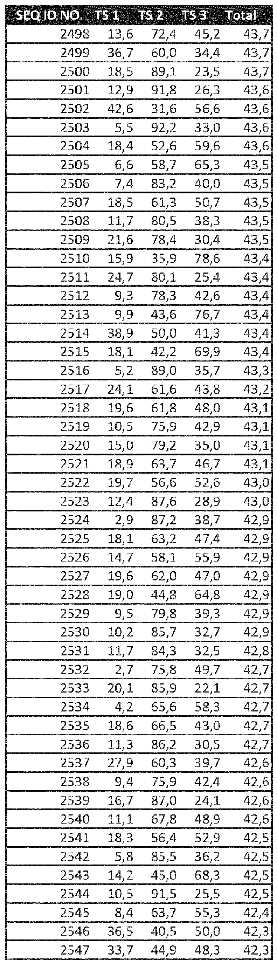

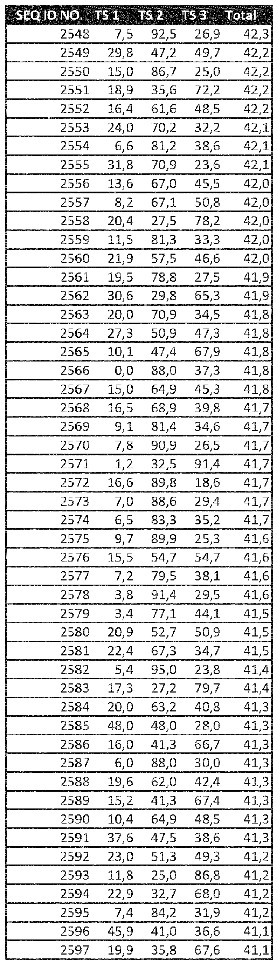

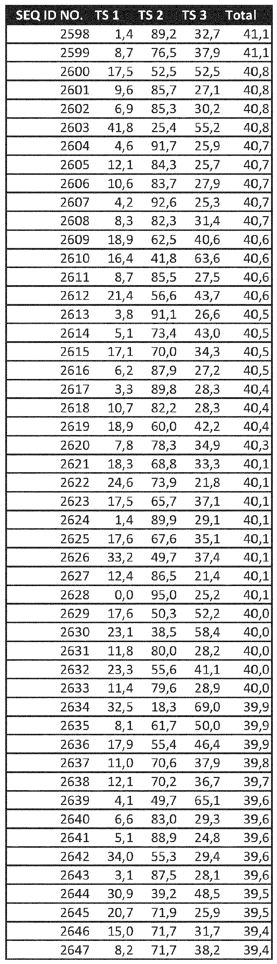

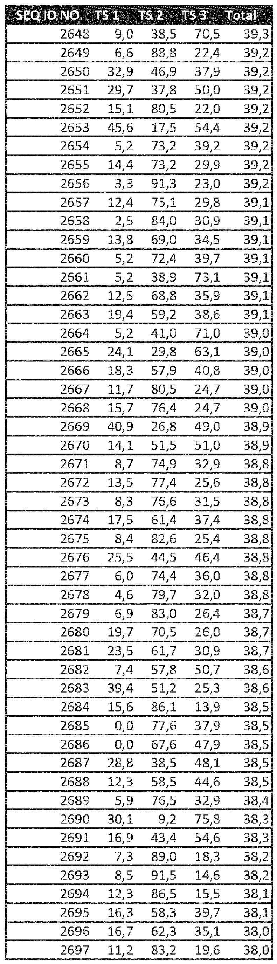

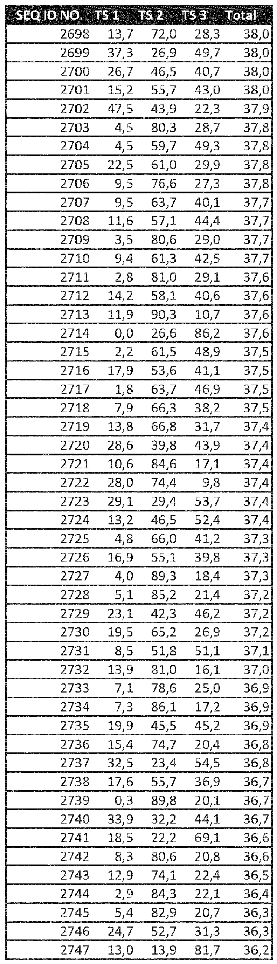

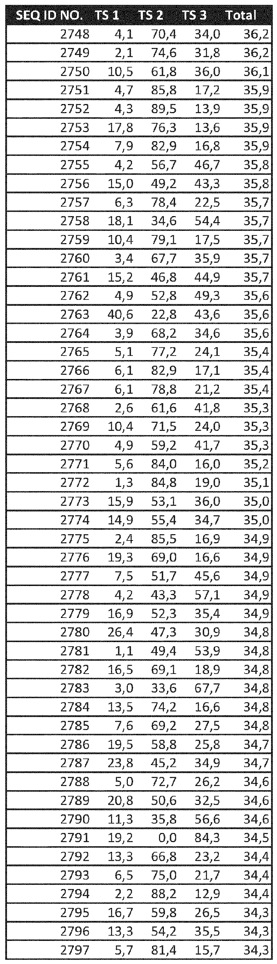

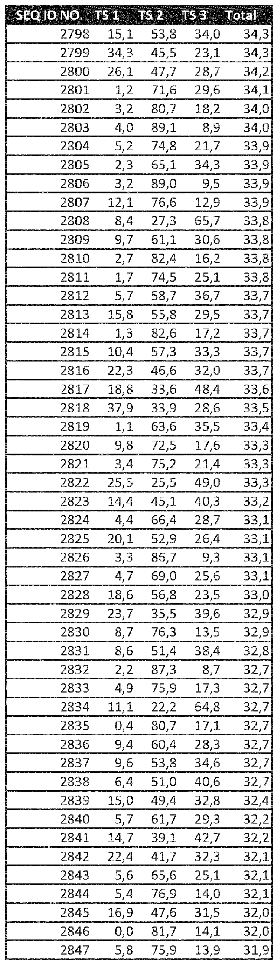

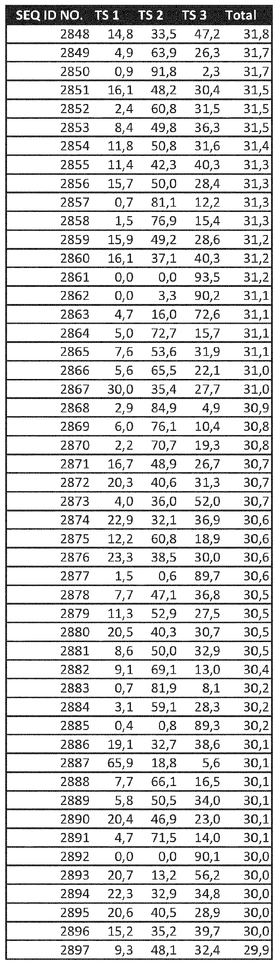

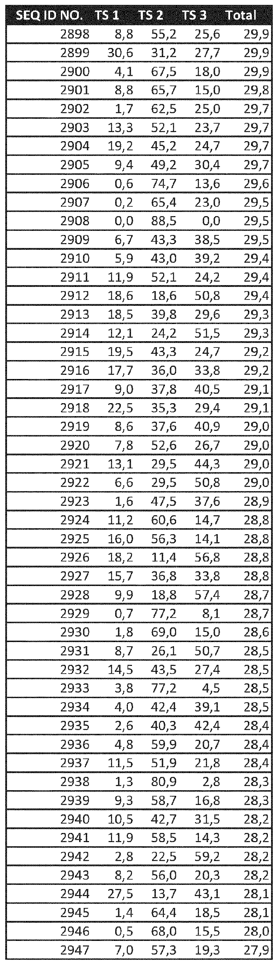

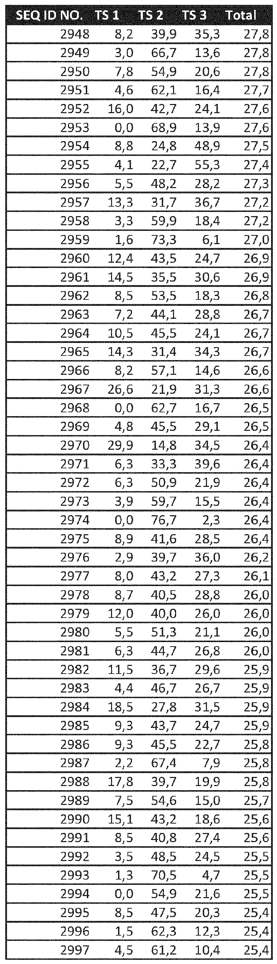

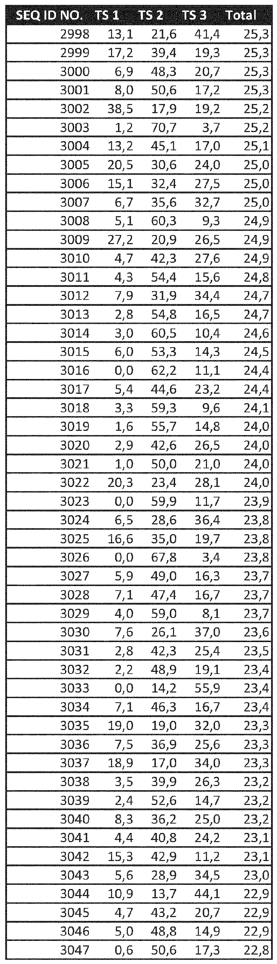

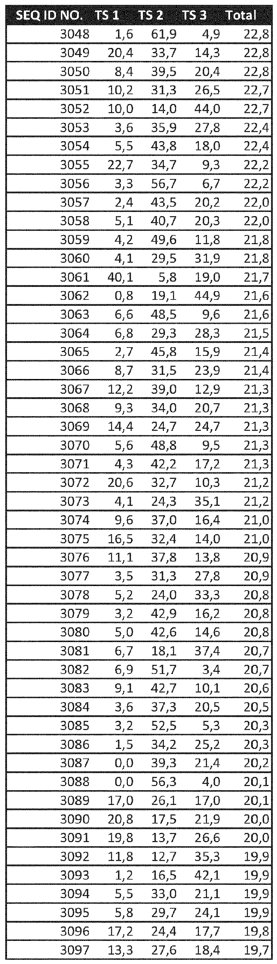

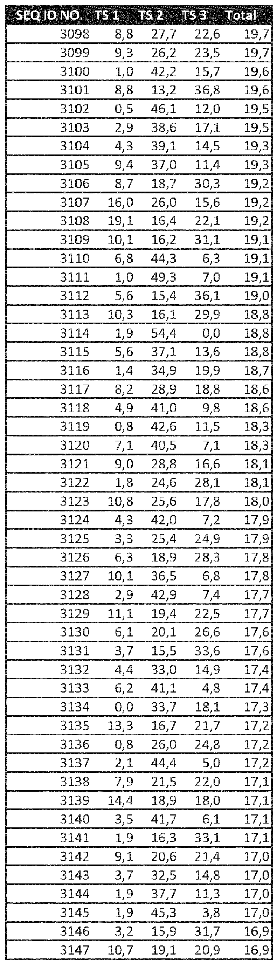

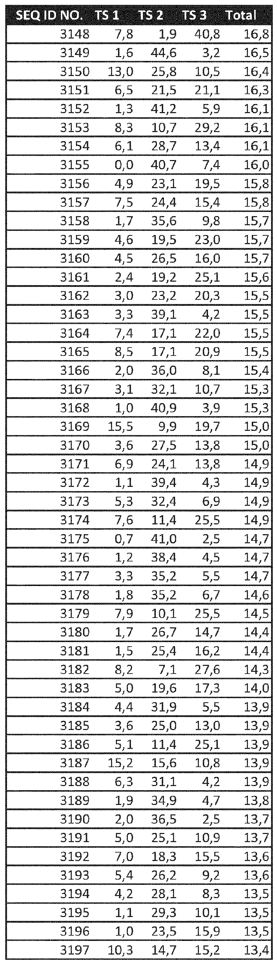

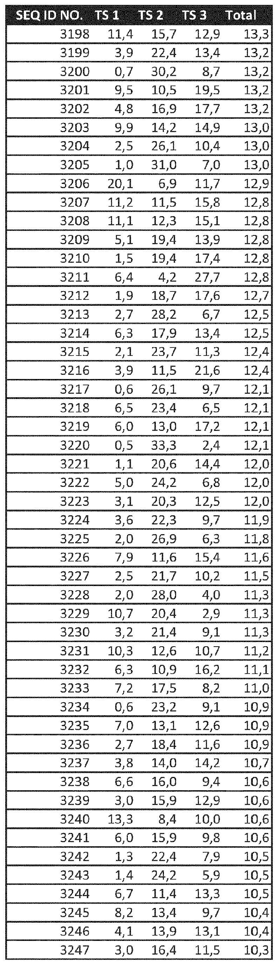

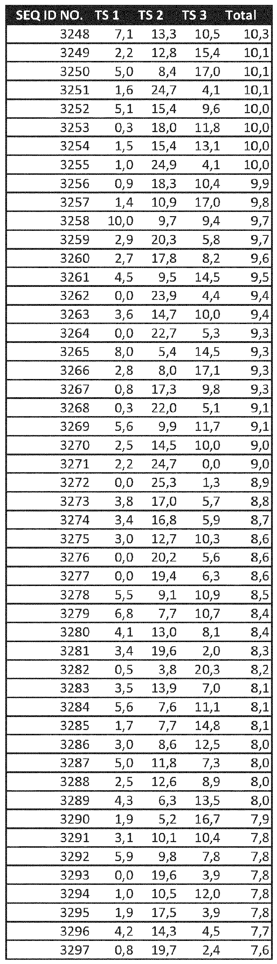

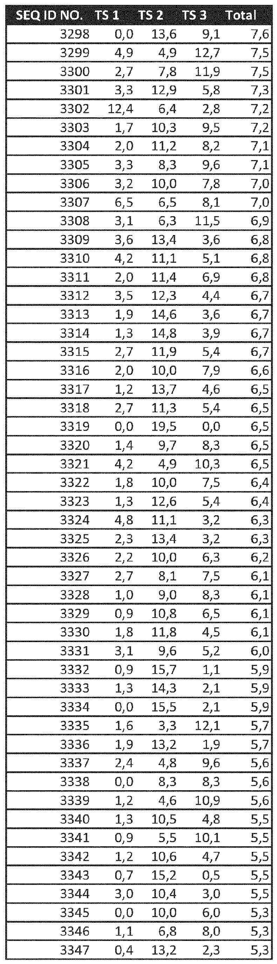

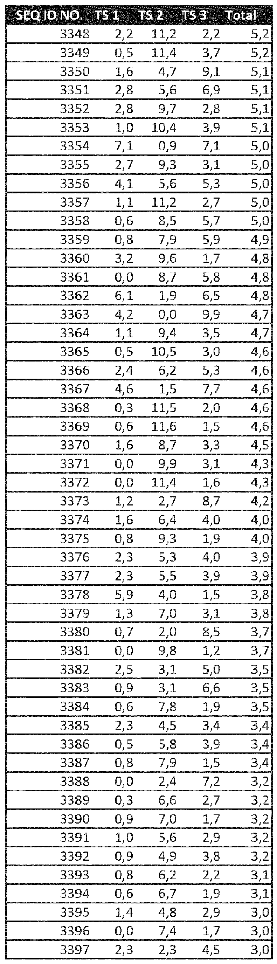

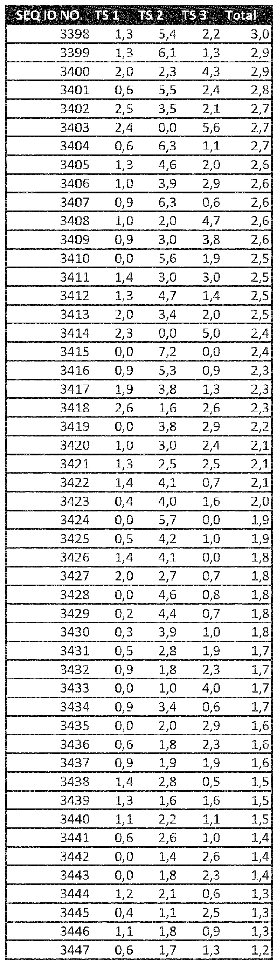

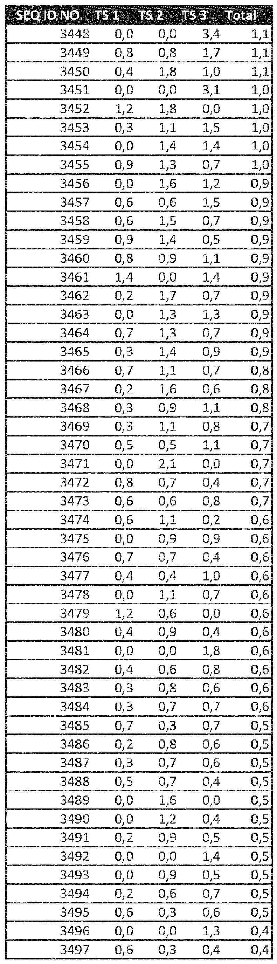

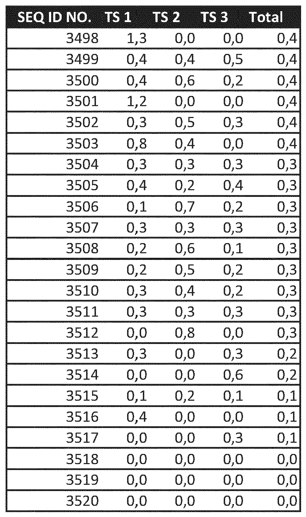

- **Fig. 2A**: is a schematic illustration of the base editing assay explained in detail in Example 8. **Fig. 2B** shows agarose gel analysis of the base editing assays performed for Cas12f-ABE (original enzyme, SEQ ID NO: 1) and for the evolved library derived from the original enzyme (WT) on target sites 1 to 3 (SEQ ID NOs: 3521, 3522 and 3523). **Fig. 2C** shows the base editing assay results for the evolved and enrich Cas12f-ABE library on different protein expression levels (induced with L-arabinose).

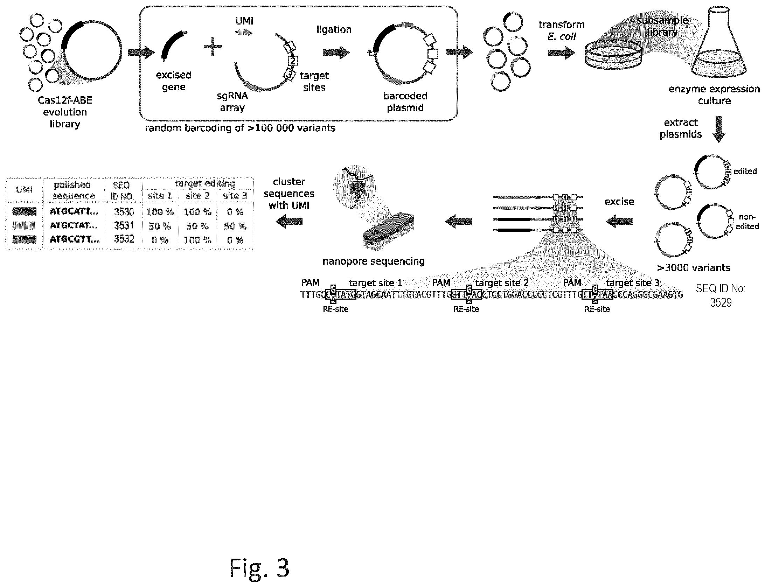

- **Fig. 3**: shows an overview of the workflow of a further particularly preferred method according to the present invention. Evolved variants of Cas12f-ABE are cloned together with a unique molecular identifier (UMI) into a vector containing three target sites. After transformation into bacteria, transformed bacteria are cultured to express the enzymes. Plasmid DNA is isolated, and the region of interest (comprising regions 1 and 2) is excised and sequenced. Clustering of the UMIs allows consensus sequence polishing and determining the base editing events.

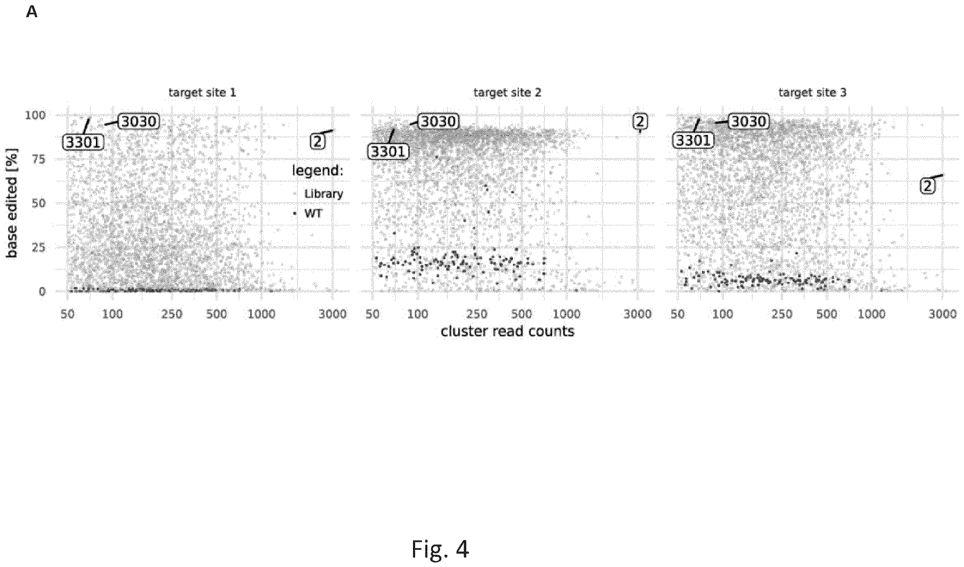

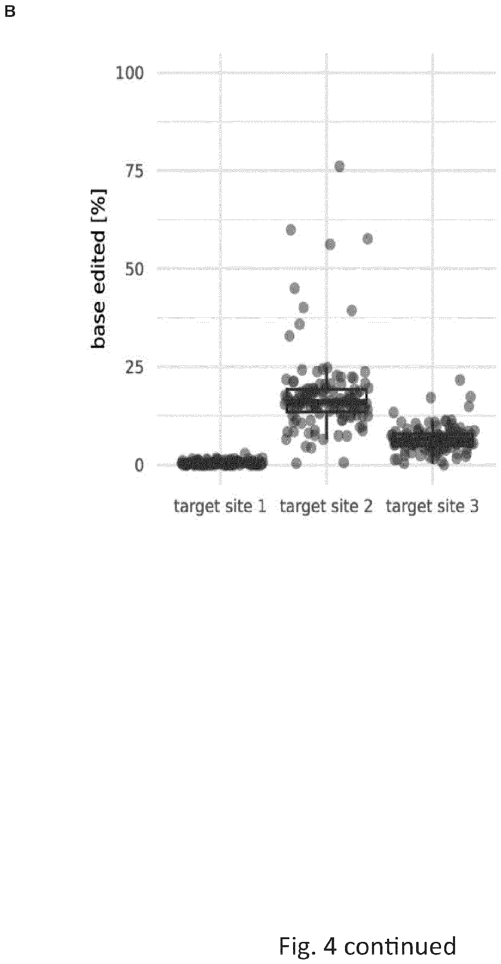

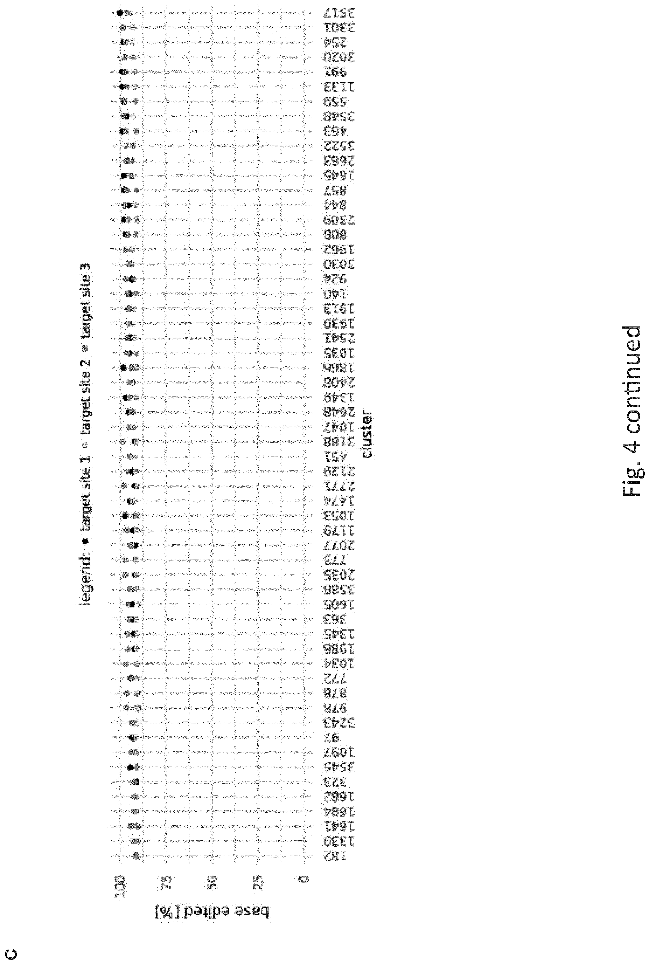

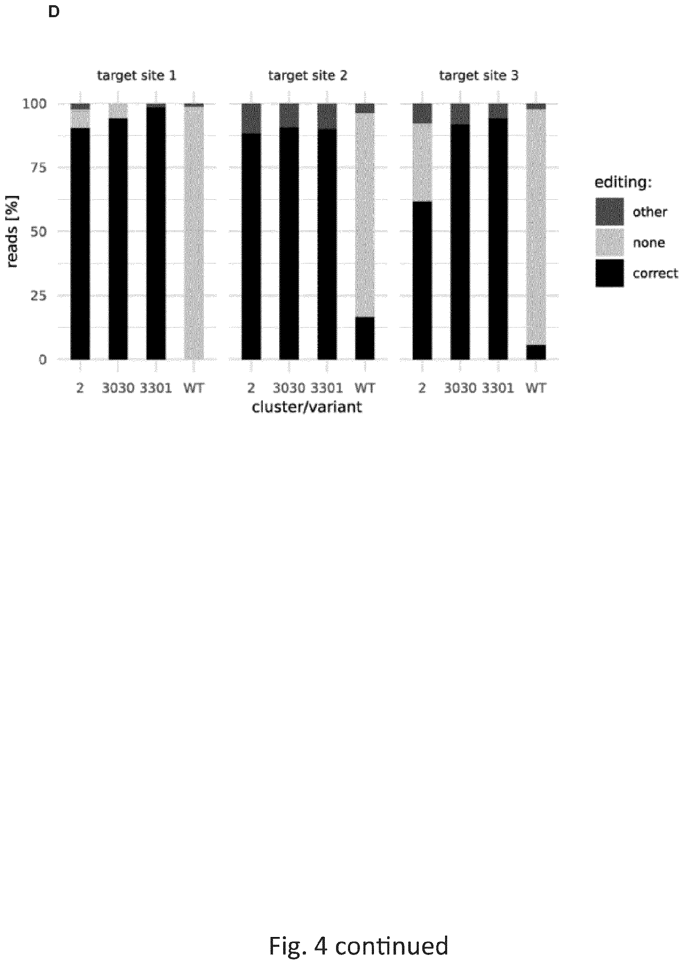

- **Fig. 4A**: shows the results of a screen of evolved variants of Cas12f-ABE on three target sites, namely target site 1 (SEQ ID NO: 3521), target site 2 (SEQ ID NO: 3522), target site 3 (SEQ ID NO: 3523). UMI-clusters containing evolved variants of Cas12f-ABE are indicated as grey spots, and original enzyme (WT, SEQ ID NO: 1) control clusters are indicated as black spots. Three selected clusters are highlighted (2 (SEQ ID NO: 471), 3030 (SEQ ID NO: 20), 3301 (SEQ ID NO: 3)) **Fig. 4B** shows median editing rates of the original enzyme clusters. **Fig. 4C** shows that 58 non-original clusters identified with the method of the present invention had editing rates of more than 90% on all three target sites. **Fig. 4D** shows the percentages of the reads of the Cas12f-ABE screen with correct editing, no editing or other editing outcomes of the original enzyme clusters (WT) and the three variants from clusters 2, 3030 and 3301 on the three target sites used in the screen (Figs. 3 and 4A, SEQ ID NOs: 3521, 3522 and 3523).

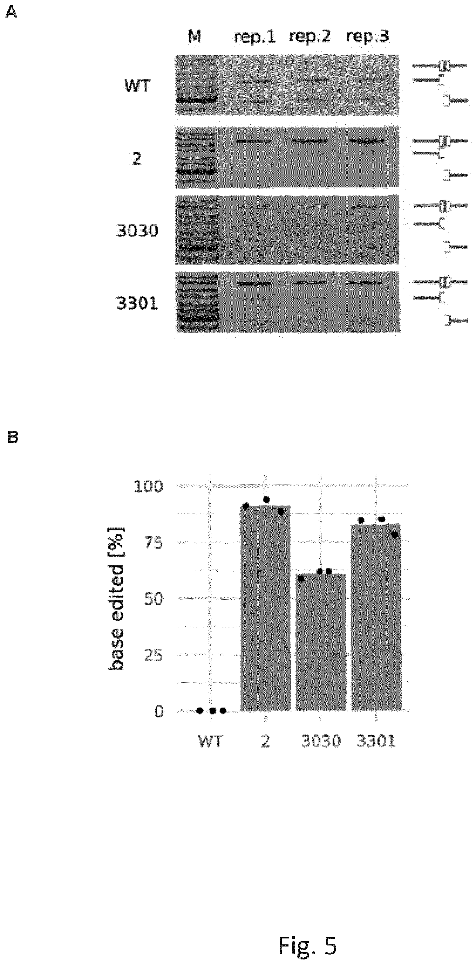

- **Fig. 5A**: shows respective agarose gel analysis of the base editing plasmid assays performed for four different DNA modifying enzymes (Clusters 2, 3030, 3301 and original enzyme (WT) control) on three different target sites simultaneously (target site 1 (SEQ ID NO: 3521), target site 2 (SEQ ID NO: 3522), target site 3 (SEQ ID NO: 3523)) in triplicate replicates (rep. 1-3). **Fig. 5B** shows the quantified results of the base editing plasmid assay (Fig. 5A). The band intensities of the edited and non-edited products of the selected Cas12f-ABE clusters (2, 3030, 3301) and the original enzyme (WT) control were determined using the image analysis software Fiji. Band intensity values of the edited products were then divided by the combined values of the edited and non-edited bands to calculate a fraction, which was converted to a percentage value. Each dot represents one replicate of the assay of the respective variant.

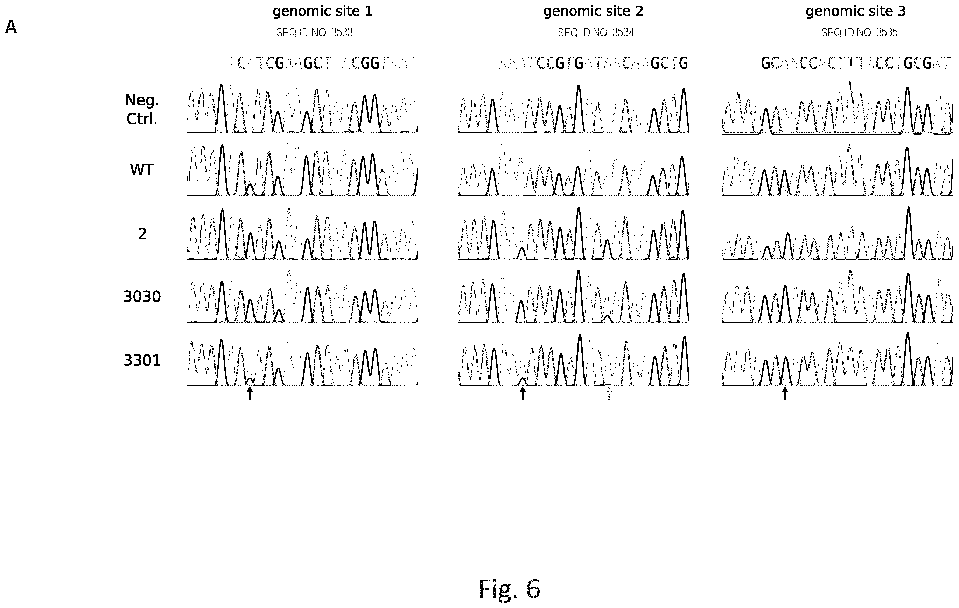

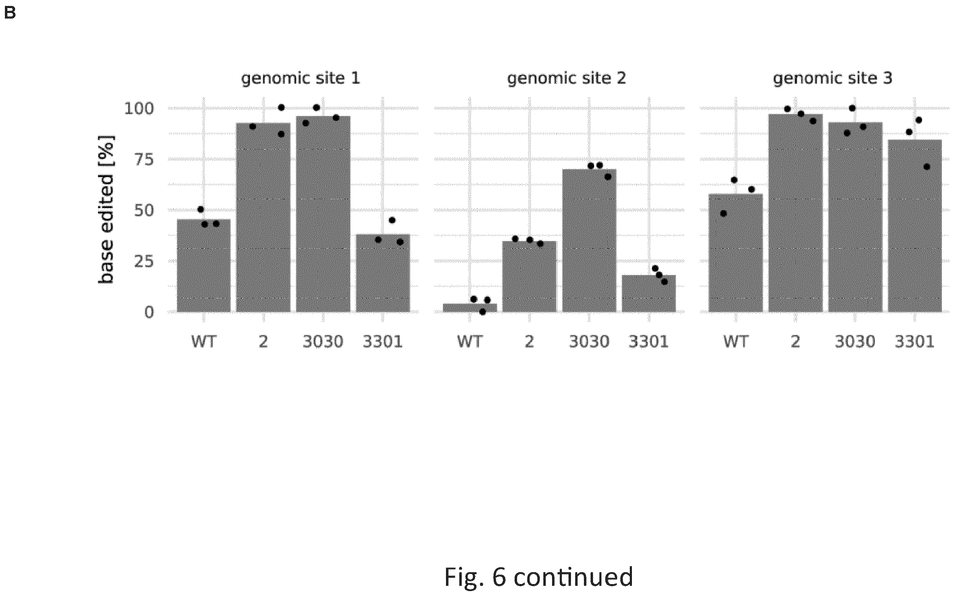

- **Fig. 6A**: shows editing results of the DNA modifying enzymes from clusters 2, 3030, 3301 and the original (WT) Cas12f-ABE on three *E. coli* genome target sites (SEQ ID NOs: 3533, 3534 and 3535) analyzed by Sanger sequencing. Included is also a negative control where no DNA modifying enzymes were expressed in the bacterium. The arrow indicates where base editing is supposed to occur. The sequence of the non-edited genomic DNA is displayed above the curves. Bases were also placed directly above each line graph at the position where editing is supposed to happen. In case of two bases at this position, the upper base corresponds to the higher curve. **Fig. 6B** shows quantified base editing rates of the Sanger sequencing results from Fig. 6A using the EditR program (Kluesner et al., 2018).

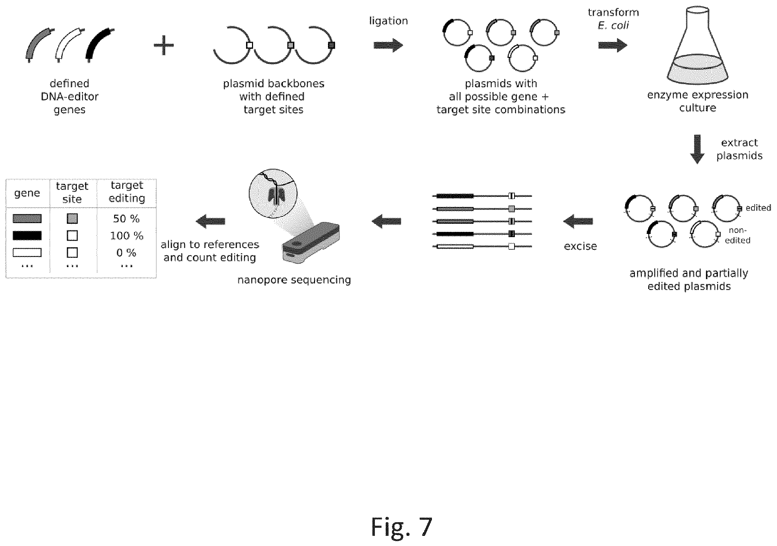

- **Fig. 7**: shows an overview of the workflow of a further particularly preferred method according to the present invention. Sequences encoding different variants of a DNA modifying enzyme are cloned into plasmids comprising respective enzyme target sites. *E. coli* cells are transformed with the vectors. Transformed bacteria are cultured to express the encoded DNA modifying enzymes. Plasmid DNA is isolated. From all plasmids isolated from the cell cultures, the region of interest (comprising regions 1 and 2) is excised and sequenced by nanopore sequencing.

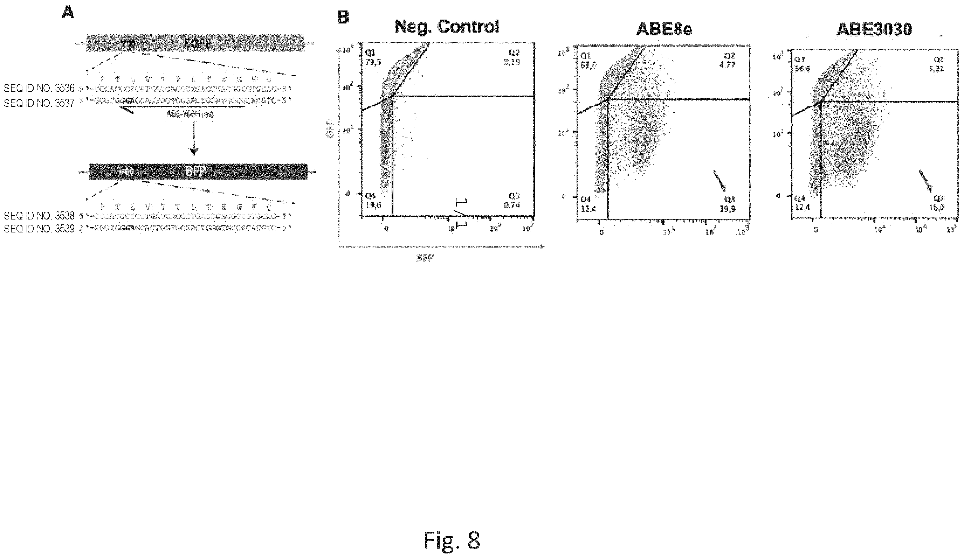

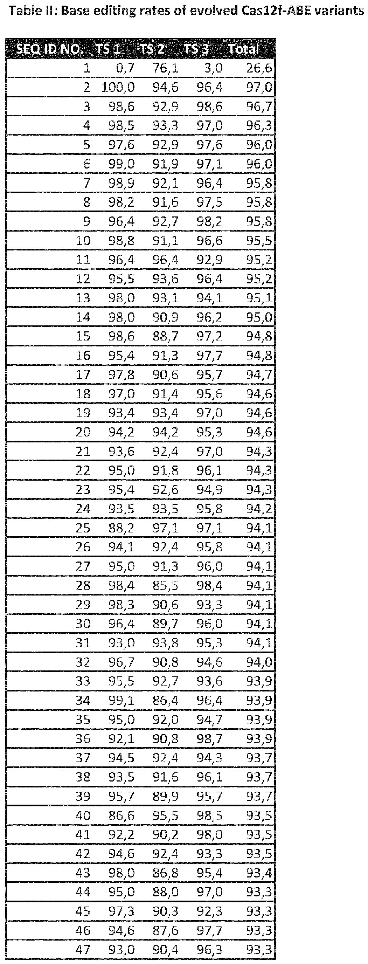

- **Fig. 8A**: shows comparative base editing efficiencies between ABE8e and ABE3030. Schematic illustration of the GFP-to-BFP conversion. The employed sgRNA is highlighted by an arrow, with the protospacer-adjacent motif (PAM) sequence shown in bold italicized and the codon to be altered shown in bold light grey. The changed amino acids are depicted, respectively. **Fig. 8B** shows FACS profiles of HEK293-eGFP cells 7 days post co-transfection with mock transfected (Neg. Control) or ABE8e and ABE3030 mRNA transfected cells in combination with GFP-sgRNA-Y66H. Note the increase in BFP converted cells for the ABE3030 transfected cells (indicated by arrows).

### Sequences

The sequences referred to in the present description are disclosed in the accompanying sequence listing.

### Detailed description of the invention

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages, different aspects of the invention are defined in more detail. Each aspect so defined may be combined with any other aspect or aspects unless clearly indicated to the contrary. Any feature indicated as being optional, preferred or advantageous may be combined with any other feature or features indicated as being optional, preferred or advantageous.

As used in this specification and the appended claims, the singular forms "a", "an", and "the" include plural referents, unless the content clearly dictates otherwise.

The term "nucleic acid" and "nucleic acid molecule" are used synonymously herein and are understood as well-accepted in the art, *i.e.* as single or double-stranded oligo- or polymers of deoxyribonucleotide or ribonucleotide bases or both. The term "nucleic acids" as used herein includes not only deoxyribonucleic acids (DNA) and ribonucleic acids (RNA), but also all other linear polymers in which the bases adenine (A), cytosine (C), guanine (G) and thymine (T) or uracil (U) are arranged in a corresponding sequence (nucleic acid sequence). The invention also comprises the corresponding RNA sequences (in which thymine is replaced by uracil), complementary sequences and sequences with modified nucleic acid backbone or 3 'or 5'-terminus. Nucleic acids in the form of DNA are however preferred.

The term "target site" (also referred to as "recognition site") as used herein refers to a specific nucleotide sequence which a DNA modifying enzyme recognizes, and at which or in the vicinity of which a DNA modification such as breakage and strand exchanges occur. One example of a target site is a target site for Cas like gene editors. The target site for the Un1Cas12f1 enzyme for example has a 4 bp PAM site (TTTR) followed by a 20 bp recognition site for the sgRNA. Further target sites of DNA modifying enzymes are e.g. Un1Cas12f1 target sites (disclosed e.g. in Xin et al., 2022), Cas9 target sites (disclosed e.g. in Cong et al., 2013), TALEN target sites (disclosed e.g. in Gaj et al., 2013), and homing endonuclease target sites (disclosed e.g. in Stoddard, 2006). All scientific literature cited herein is incorporated by reference.

The term "unique molecular identifier" ("UMI") as used herein denotes a type of molecular barcoding. Molecular barcodes are short sequences to uniquely tag a molecule in a sample library. UMIs are known to the skilled person and are described in detail in Zurek et al., 2020, or Karst et al., 2021, both of which are herein incorporated by reference in their entirety. According to a preferred embodiment of the present invention, a UMI is an oligonucleotide comprising at least 5, at least 6, at least 7, at least 8, at least 9, at least 10, at least 20, at least 30, at least 40, at least 50, at least 60, at least 70, at least 80, at least 90 or at least 100 random nucleotides. According to a particularly preferred embodiment, a UMI is an oligonucleotide comprising at least 50 random nucleotides. A UMI may preferably form part of a UMI-tag, which may further comprise one or more sequences down-stream and/or upstream of the random nucleotides of the UMI (e.g. flanking the random nucleotides), which sequences may serve as one or more primer binding sites. A UMI-tag may further comprise one or more and preferably at least two restriction sites preferably down-stream and/or upstream of the random nucleotides of the UMI. According to a preferred embodiment, a UMI-tag comprises a first primer binding site, a first restriction site, the random nucleotides of the UMI, a second restriction site, and a second primer binding site.

The term "cell" or "host cell" as used herein relates to an intact cell, *i.e.* a cell with an intact membrane that has not released its normal intracellular components such as enzymes, organelles, or genetic material. An intact cell preferably is a viable cell, *i.e.* a living cell capable of carrying out its normal metabolic functions. Preferably said term relates to any cell which can be transfected or transformed with an exogenous nucleic acid.

The term "regulatory nucleic acid sequence" as used herein refers to gene regulatory regions of DNA. In addition to promoter regions, this term encompasses operator regions more distant from the gene as well as nucleic acid sequences that influence the expression of a gene, such as cis-elements, enhancers or silencers. The term "promoter region" as used herein refers to a nucleotide sequence on the DNA allowing a regulated expression of a gene. The promoter region allows regulated expression of the nucleic acid encoding for the respective protein. The promoter region is located at the 5'-end of the gene and thus before the coding region. Both, bacterial and eukaryotic promoters are applicable for the present invention.

The term "identical" is used herein in the context of two or more nucleic acids or polypeptide sequences, to refer to two or more sequences or subsequences that are the same, *i.e.* that comprise the same sequence of nucleotides or amino acids. Sequences are "identical" to each other if they have the same sequence of nucleotides or amino acid residues (= 100% identity). The term "essentially identical" used in the context of two nucleic acids or polypeptides means that the two sequences compared share at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identity over the specified sequence, when compared and aligned for maximum correspondence over a comparison window, or designated region as measured using one of the following sequence comparison algorithms or by manual alignment and visual inspection. The percentage identity for "essentially identical" likewise applies for sequences that are "essentially reverse complementary" to each other.

### Description of embodiments

According to a first aspect, the invention provides a DNA modifying enzyme comprising or consisting of a DNA editing protein and a Cas protein and a peptide linker,
wherein said base editing protein and said Cas protein are connected by said peptide linker; and
wherein said DNA modifying enzyme has been obtained by directed molecular evolution of an original enzyme; and
wherein said DNA modifying enzyme shows an editing rate which is at least 10%, 20% or 30 %, preferably at least 40 %, more preferably at least 50 %, most preferably at least 60 % higher than the base editing rate of said original enzyme.

Said DNA modifying enzyme preferably shows an editing rate which is at least 30 %, preferably at least 40 %, more preferably at least 50 %, most preferably at least 60 % higher, or even 70%, 80% or 90 % higher than the editing rate of the original enzyme on at least one target site, preferably on two target sites, more preferably on three target sites of said DNA modifying enzyme.

"Original enzyme" in accordance with the invention means the DNA modifying enzyme variant that is used as starting material for the directed molecular evolution. The DNA modifying enzyme can be a naturally occurring DNA modifying enzyme or it can be a variant of a naturally occurring DNA modifying enzyme. According to a preferred embodiment of the present invention, the DNA modifying enzyme is an evolved enzyme. Preferably, the DNA modifying enzyme has been evolved applying Cas-assisted substrate linked directed evolution (CaSLiDE). SLiDE has been described in e.g. Buchholz and Stewart 2001, Buchholz and Hauber 2011, Lansing et al. 2019, Hoersten et al. 2021, and Lansing et al. 2022, and is applicable to site-specific recombinases. However, it cannot be applied to CRISPR-Cas systems. The invention to develop Cas-assisted SLiDE (CaSLiDE) is described in examples 4 and 9 hereinbelow. As such, "directed molecular evolution" in accordance with the invention is preferably Substrate Linked Directed Evolution (SLiDE) as known in the art or which has been adapted as detailed herein in the second aspect of the invention and in examples 4 and 9 hereinbelow.

In accordance with the present invention and as defined above, a "target site" of a DNA modifying enzyme is a nucleotide sequence. According to a preferred embodiment, the target site can be the target site known for the respective DNA modifying enzyme. However, the target site is not limited to the specific target site that might be known in the art for the DNA modifying enzyme. The target site can be a modified version of a known target site. As such, the target site can be a naturally occurring target site of a DNA modifying enzyme, or it can be an artificially created target site such as a modified version of a known target site, preferably of a known naturally occurring target site. Such modified versions of a known target site preferably differ in one, two, three, four, five, six, seven, eight, nine, ten, eleven or twelve nucleotides from the known target site. A modified target site may alternatively differ from a known target site in about 2%, about 4%, about 6%, about 8%, about 10%, about 12%, about 14%, about 16%, about 18%, about 20%, about 25%, about 30%, about 35%, about 40%, about 45%, or in about 50% of the nucleotides.

Said DNA editing protein as part of the DNA modifying enzyme of the invention is preferably selected from the group consisting of deoxycytidine deamination-derived editors (CBEs) which facilitate C•G to T•A mutations, deoxycytidine deamination-derived editors (CGBEs) which facilitate C•G to G•C mutations, deoxyadenosine deamination-derived base editors (ABEs) which facilitate A•T to G•C mutations, and prime editing enzymes such as reverse transcriptases in combination with a prime editing guide (peg)RNA, which facilitate all types of DNA substitution, insertions and deletions on a target site.

In a more preferred embodiment, the DNA modifying enzyme of the invention is a deoxycytidine deamination-derived editor (CBE).

In an even more preferred embodiment, the DNA modifying enzyme of the invention is a deoxyadenosine deamination-derived base editor (ABE).

"DNA editing" generally means any type of DNA substitution, insertions and deletions on a target site. In preferred embodiments, the term "DNA editing" relates to "base editing", i.e. base exchanges of DNA and "DNA editing rate" relates to "base editing rate" of DNA.

Said Cas protein as part of the DNA modifying enzyme of the invention is preferably selected from the group consisting of Cas9; Cas12, such as Cas 12a, Cas 12b, Cas 12c, Cas12d, Cas12f, Cas12i, Cas12j, Cas12g, Cas12h, TnpB, Casµ; and Cas13, such as Cas13a, Cas13b, Cas13c, Cas13d. Most preferably, said Cas protein is Cas12f.

Thus, in a preferred embodiment of the invention, said DNA modifying enzyme comprises or consists of a base editing protein, preferably an ABE base editor protein, a Cas 12f protein and a peptide linker.

Said peptide linker typically comprises or consists of 5 to 35 amino acids, preferably of 32 amino acids.

Surprisingly, the inventors have overcome the obstacles of the prior art and have generated a huge number of DNA modifying enzymes comprising or consisting of an ABE base editor protein, a Cas 12f protein and a peptide linker, namely the DNA modifying enzymes of SEQ ID Nos. 2 to 3520. Thereby, the efficacy of the DNA modifying enzymes could be improved in all three parts of the DNA modifying enzymes, i.e. in the ABE base editor protein, the peptide linker and in the Cas12f protein.

Accordingly, in a preferred embodiment of the invention, said ABE base editor protein is selected from the group consisting of proteins consisting of amino acids 1 to 166 of SEQ ID NOs. 2 to 3520. Said ABE base editor protein preferably comprises at least one single amino acid substitution, preferably wherein the single amino acid substitution is comprised in the catalytic region, preferably wherein the single amino acid substitution is at a position of a conserved amino acid in the catalytic region of said ABE base editor, when compared to the original ABE base editor protein, which has the amino acid sequence of amino acids 1 to 166 of SEQ ID NO. 1.

In a further preferred embodiment of the invention, said Cas12f protein is selected from the group consisting of amino acids 199 to 726 of SEQ ID NOs. 2 to 3520. Said Cas12f nuclease preferably comprises at least one single amino acid substitution, preferably wherein the single amino acid substitution is comprised in the catalytic region, preferably wherein the single amino acid substitution is at a position of a conserved amino acid in the catalytic region of said Cas 12f protein, when compared to the amino acid sequence of the original Cas 12f protein, which has the amino acid sequence of amino acids 199 to 726 of SEQ ID NO. 1.

Further preferably, said peptide linker is selected from the group consisting of amino acids 167 to 198 of SEQ ID NOs. 2 to 3520 and comprises at least one single amino acid substitution, when compared to the amino acid sequence of the original linker, which has the amino acid sequence of amino acids 167 to 198 of SEQ ID NO. 1.

Amino acid substitutions in the amino acid sequence of SEQ ID NO. 1, which lead to superior base editing efficacy of the DNA modifying enzyme of the invention are selected from those listed in Table I. These amino acid substitutions occurred in 30 % of the 30 DNA modifying enzymes with the highest base edition rates provided by the invention.

**Table I: Amino acid substitutions which lead to superior base editing efficacy of the DNA modifying enzyme of the invention.**

| **Amino acid position no.** | **Amino acid in original enzyme (SEQ ID NO. 1)** | **Exchanged by** |
|---|---|---|
| 32 | V | I |
| 75 | I | T/A/V |
| 110 | R | H |
| 128 | R | C |
| 137 | A | V/M |
| 155 | F | L |
| 158 | Q | R |
| 166 | N | S/R |
| 182 | T | A/P |
| 186 | A | V/P/T |
| 189 | E | K/G/V |
| 196 | G | A/S/T |
| 249 | K | E |
| 255 | A | S/T |
| 295 | I | L/T |
| 296 | S | G |
| 328 | I | F |
| 361 | S | G/N |
| 363 | I | V |
| 406 | N | S |
| 409 | S | D/G |
| 441 | Q | R |
| 473 | K | R |
| 538 | F | L/S |
| 550 | H | R |
| 583 | T | S/A |
| 590 | E | K/R/G/V |
| 659 | E | K/V |
| 705 | N | D |

Accordingly, the invention provides in a preferred embodiment DNA modifying enzymes comprising single amino acid substitutions at least at one or at more positions selected from the group consisting of those listed in table I.

The DNA modifying enzymes of the invention comprising or consisting of an ABE base editor protein, a Cas 12f protein and a peptide linker, namely the DNA modifying enzymes of SEQ ID Nos. 2 to 3520, show base editing rates on at least one of the target sites selected from the group consisting of target site 1 (SEQ ID NO: 3521), target site 2 (SEQ ID NO: 3522), target site 3 (SEQ ID NO: 3523), preferably an editing activity on each of target site 1 (SEQ ID NO: 3521), target site 2 (SEQ ID NO: 3522), and target site 3 (SEQ ID NO: 3523) of more than 70 %. (preferably of more than 80 %, more preferably of more than 90 %, most preferably of more than 95%).

Accordingly, the present invention provides in a further embodiment a DNA modifying enzyme which shows an editing activity on each of target site 1 (SEQ ID NO: 3521), target site 2 (SEQ ID NO: 3522), target site 3 (SEQ ID NO: 3523), wherein said DNA modifying enzyme is selected from the group consisting of SEQ ID NO. 2 to 3520.

Further preferably, the DNA modifying enzyme shows an editing activity on all three of target site 1 (SEQ ID NO: 3521), target site 2 (SEQ ID NO: 3522), target site 3 (SEQ ID NO: 3523) of equally to or more than 90 %, and wherein said DNA modifying enzyme is selected from the group consisting of SEQ ID NOs. 2, 3, 4, 5, 6, 7, 8, 9, 10, 11, 12, 13, 14, 16, 17, 18, 19, 20, 21, 22, 23, 24, 26, 27, 29, 30, 31, 32, 33, 35, 36, 37, 38, 39, 41, 42, 45, 47, 48, 49, 50, 51, 54, 56, 57, 60, 61, 63, 64, 67, 69, 70, 73, 75, 76, 77, 78, 79, 82, 83, 84, 87, 88, 89, 91, 92, 93, 94, 100, 104, 106, 107, 110, 111, 114, 116, 120, 124, 134, 135, 138, 143, 154, 165.

Most preferably, the DNA modifying enzyme of the invention belongs to the 30 DNA modifying enzymes with the highest editing activity provided herein and is selected from SEQ ID NOs. 2, 3, 4, 5, 6, 7, 8, 9, 10, 11, 12, 13, 14, 15, 16, 17, 18, 19, 20, 21, 22, 23, 24, 25, 26, 27, 28, 33, 101, 471,

According to the second aspect, the invention provides a method of directed molecular evolution of a DNA modifying enzyme or of any of the accompanying factors (or components) such as sgRNA scaffold, or pegRNA scaffold in the case of prime editing, said method as described hereinbefore, said method comprising the steps of
a. Generation of a library of said DNA modifying enzyme,
b. Cloning said library in expression vectors containing the nuclei acid of at least one target site, preferably of 2 or 3 target sites of said DNA modifying enzyme,
c. Transformation of the expression vector of step b. into a host cell and expression of the library of said DNA modifying enzyme in said host cell;
d. Isolation of plasmids and digestion with the restriction enzymes, which target the nucleic acids of said target sides;
e. Selection of linearized plasmids containing a DNA edition in the nucleic acid of at least one target site, preferably 2 or 3 target sites,
f. Subjecting the linearized plasmids to a PCR or DNA shuffling and repeating steps b. to e. to enrich the library of DNA modifying enzymes.

Preferably, said library of a DNA modifying enzyme that is generated in step a) is a library of DNA modifying enzymes comprising or consisting of an ABE base editor protein, a Cas12f protein and a peptide linker (a Cas12f-ABE library). As described in the examples below, said library of DNA modifying enzymes may be generated using error-prone-PCR, e.g. with a low-fidelity DNA polymerase.

The expression vector in accordance with the present invention is not limited to any specific expression vector. Typically, an expression vector comprises an origin of replication, a promoter, as well as specific gene sequences that allow phenotypic selection of host cells comprising the vector. In accordance with the present invention, the vector comprises a first region encoding a variant of a DNA modifying enzyme, and a second region comprising at least one, at least two, at least three or more target sites of a DNA modifying enzyme and as defined herein above.

A particularly preferred expression vector to be used in accordance with the present invention is the pEVO vector as described in Buchholz and Stewart 2001.

In cases of using evolved DNA modifying enzymes in the method of the present invention, the gene encoding the DNA modifying enzyme is preferably excised from a respective evolution library as shown in Fig. 3. Respective evolution libraries are described e.g. in Buchholz and Stewart 2001, and in Lansing et al. 2022.

According to the present invention, the library of expression vectors is introduced into the host cells. A host cell within the meaning of the invention is a naturally occurring cell or a cell line (optionally transformed or genetically modified) that comprises at least one vector as described above. The invention thus includes host cells that include at least one expression vector according to the invention as a plasmid. According to a preferred embodiment, a host cell includes one expression vector according to the invention as a plasmid. This embodiment is particularly useful if bacteria are used as host cells.

According to an embodiment of the present invention, the host cell is selected from the group consisting of bacterial cells, yeast cells, fungal cells, and mammalian cells. Suitable bacterial cells include cells from gram-negative bacterial strains such as strains of *Escherichia coli, Proteus,* and *Pseudomonas,* and gram-positive bacterial strains such as strains of *Bacillus, Streptomyces, Staphylococcus,* and *Lactococcus.* Suitable fungal cells include cells from species of *Trichoderma, Neurospora,* and *Aspergillus.* Suitable yeast cells include cells from species of *Saccharomyces* (for example *Saccharomyces cerevisiae*), *Schizosaccharomyces* (for example *Schizo saccharomyces pombe*), *Pichia* (for example *Pichia pastoris* and *Pichia methanolicd*), and *Hansenula.* Suitable mammalian cells include for example CHO cells, BHK cells, HeLa cells, COS cells, HEK293T and the like. However, amphibian cells, insect cells, plant cells, and any other cells used in the art for the expression can be used as well. According to a preferred embodiment, the host cell is a bacterial cell. According to a particularly preferred embodiment, the host cell is an *E. coli* cell.

The introduction of the nucleic acids into the host cells is performed using techniques of genetic manipulation known by a person skilled in the art. Among suitable methods are cell transformation, transfection or viral infection, whereby a nucleic acid sequence encoding the DNA modifying enzyme is introduced into the cell as a component of the vector. The cell culturing is carried out by methods known to a person skilled in the art for the culture of the respective cells. Therefore, cells are preferably transferred into a conventional culture medium, and cultured at temperatures and in a gas atmosphere that is conducive to the survival of the cells and allows expression of the encoded protein. A preferred method for introducing the library of expression vectors into host cells is transformation, preferably by electroporation. As an example of the step of introducing the library of expression vectors into host cells, E. coli cells can be transformed with the vectors using electroporation.

According to one embodiment of the method of the present invention, the host cells comprising the expression vectors are plated on a suitable medium such as an agar plate to allow growing and selection of individual cell colonies. This step may also be used for selecting a specific number of individual colonies instead of culturing all cells simultaneously, thereby decreasing the overall number of the members of the library and reducing the number of enzyme variants to be screened. Alternatively, the host cells comprising the expression vectors can be briefly cultured in a respective culture medium (e.g. for a limited time, for example 0.5 to 1 hour) and an amount of the culture medium equal to the desired number of variants can be taken from this culture medium for further culturing as described in the following. The number of transformed bacteria present per µl of culture medium can be estimated based on the number of colonies on the plates. If for example 10 µl of the medium were spread on a plate and the plate after incubation shows 100 distinct colonies, one µl of medium contains ten transformed bacteria. According to the present invention, the host cells comprising the library of expression vectors or the cells selected as explained above are cultured to allow the expression of the plasmid. Culturing conditions depend on the cell line used and are not particularly limited. It is, however, not necessary to culture each selected colony individually. The selected colonies can be cultured together in a single cell culture. Alternatively, the host cells comprising the library of expression vectors or the cells selected as explained above are cultured in more than one separate cell culture. For activation of the expression of the nucleic acid encoding for the DNA modifying enzyme, the nucleic acid encoding for the DNA modifying enzyme further comprises a regulatory nucleic acid sequence, preferably a promoter region. Hence, expression of the nucleic acid encoding for DNA modifying enzyme is initiated or regulated by activating the regulatory nucleic acid sequence. The cells are preferably cultured under conditions allowing cell growth and protein expression. Preferably, the cells are grown for more than two hours up to 12 hours or longer, allowing more than two, preferably more than three, more than four, more than five, more than six, more than seven, more than eight, more than nine or more than ten cell doublings.

Upon expression of the DNA modifying enzymes in the culture of host cells, the DNA modifying enzymes start modifying the DNA at the one or more target sites in the expression vector, if said DNA modifying enzymes recognizes the respective target site(s). After expression of the base editors, plasmids are isolated and digested with restriction enzymes, which target the target sites. Applying a restriction digest to the plasmids results in one linear fragment for the edited plasmids and in two fragments for non-edited plasmids. An error-prone PCR using the indicated primers (arrows) will exclusively generate a product of the edited plasmids. These amplified and mutated active Cas12f1-ABE variants are then subjected to the next evolution cycle.

The expression of said DNA modifying enzymes in said host cell is usually performed in presence of L-arabinose. For increasing selection pressure, L-arabinose levels and thus induction of enzyme expression are lowered from 200 µg/ml (in the initial evolution cycle) to 1 µg/ml in further evolution cycles.

In order to achieve a sufficient number of mutated DNA modifying enzymes, the method of the invention may be performed such that it includes 2 to 100, such as 10, 20, 30, 40, 50, 60, 70, 80, 90 or 100 cycles of directed molecular evolution.

In further embodiment of the method of the invention, said library of said DNA modifying enzyme may be further enriched by high fidelity PCR.

In a further embodiment of the method of the invention, said further enriched library of said DNA modifying enzyme may be screened for the DNA editing rate, preferably the base and/or prime editing rate of the DNA modifying enzymes on at least one, preferably 2 or 3 target sites, wherein said screening comprising sequencing of the DNA modifying enzymes contained in said library and activity testing in a base editing assay in comparison to the original enzyme of said DNA modifying enzyme.

To determine if any modification in the target sites of the vector has occurred, plasmid DNA is isolated from the culture(s) of host cells after culturing of the host cells and expressing the encoded library of DNA modifying enzymes. Isolating of plasmid DNA from a culture of cells can be done by any conventional methods well known in the art. After isolation, the plasmid DNA is sequenced to determine its nucleotide sequence at least in the region coding for the DNA modifying enzyme (first region) and in the region coding for the target site(s) (second region). It will be appreciated that more parts or all of the plasmid can be sequenced than regions containing the sequences encoding the one or more DNA modifying enzyme and the one or more target sites. However, for a high-throughput screening, it is preferred that the first and the second region are sequenced since the information obtained by sequencing the first and the second region is sufficient for the method of the present invention. This sequencing step allows determining the sequence of the specific DNA modifying enzyme, the sequence of the target region and whether not the DNA sequence in the second region on the expression vector has been altered by the DNA modifying enzyme. According to a preferred embodiment, the first and the second region of the vector are sequenced in a single method step, i.e. they are sequenced together and not subsequently.

The sequencing method is not particularly limited. However, for particularly high-throughput methods, long-read sequencing methods are preferred. A particularly preferred sequencing method in accordance with the present invention is nanopore sequencing as described in Wang et al., 2021 (incorporated herein by reference), or as provided by e.g. Oxford Nanopore Technologies, UK. In brief, nanopore sequencing works by monitoring changes to an ionic current as nucleic acids are passed through a protein nanopore. The resulting signal is decoded to provide the specific DNA sequence. Based on the sequencing results, an activity rate for each DNA modifying enzyme on the respective target site(s) can be determined.

In cases in which the sequences of the DNA modifying enzymes are known, sequence comparisons of the screen sequence data to the known sequences of the DNA modifying enzymes are performed to identify which sequence reads belong to which DNA modifying enzyme. DNA modifying enzyme sequences can be known because they have been sequenced previously, or because they were synthesized according to a defined sequence. The sequence reads are separated according to their sequence matches and from here on processed separately for consensus sequence generation and determination of the activity rate. It is preferred that in such cases no UMI and no UMI-tag are used in the method of the invention.

In cases in which a UMI or a UMI-tag is used, the method according to the present invention may further comprise the steps of clustering the unique molecular identifiers, generating and polishing consensus sequences, determining the number of DNA modification events for each DNA modifying enzyme, and determining an activity rate for each DNA modifying enzyme. Clustering and polishing of respective sequencing results are described in Zurek at el., 2020, and Karst et al., 2021, both of which are incorporated herein by reference in their entirety. For example, the UMI sequence of the sequencing reads can be identified by comparing the sequences upstream and/or downstream of the UMI. In case of using the upstream sequence for the identification of the UMI, the DNA sequence with the length of the UMI after the match is collected with the sequence read ID. In case of using the downstream sequence for the identification of the UMI, the DNA sequence with the length of the UMI before the match is collected with the sequence read ID. In case of using the downstream and upstream DNA for the identification of the UMI, the DNA sequence between the matches is collected with the sequence read ID. The collected UMI sequences are then clustered based on sequence identity, while maintaining their association to their read IDs, for example with vsearch (Rognes et al., 2016). Based on these clusters and their associated read IDs, the sequence reads are then separated into different files, where each file contains reads that corresponds to one UMI-cluster. From each of these clusters of separated reads, the part of the sequence that contains the gene for the DNA modifying enzyme is aligned to a reference gene that contains a high similarity to the screened variants. Typically, such a gene is determined through sequencing of the screened DNA modifying enzyme library or by selecting the DNA modifying enzyme gene that was modified to produce the screened variant library. With the aid of this alignment, a software for consensus sequence generation and sequence polishing can be used to determine the most likely gene sequence of the DNA modifying enzyme associated to this cluster based on all sequence reads. Polishing in this respect refers to additional rounds of processing of the reads, leading to improved sequence accuracy.

The method for determining the activity rate is not particularly limited. For example, the sequencing reads, which have been separated through sequence comparison or UMI clustering, are compared to expected sequences from the second region in the changed and non-changed sequences. Based on this sequence comparison, the number of reads matched to the changed DNA can be counted. The counted changes divided by the total number of reads matching the second region gives rise to an activity rate of the DNA modifying enzyme which is associated to the separated reads.

In cases in which a DNA modifying enzyme is screened on multiple target sites, additional references for the second region can be provided to associate the activity rate for the identified DNA modifying enzyme to multiple target sites. The additional references are sequences of the second region with the target sites replaced with the alternative target sites that were used in the screen. These references can also be provided in a changed and non-changed version to determine the activity rate as described above.

Genes encoding DNA modifying enzymes are cloned together with a unique molecular identifier (UMI) into an expression vector, which further contains one or more of the target sites of interest. The barcoded plasmids are transformed into respective cells, and the cells are spread on one or more culture plates. Based on the number of colonies on the plates, a defined number of clones is cultured in medium that induces the expression of the genes encoding the DNA modifying enzymes. This results in multiple copies of the transformed plasmids, with a fraction of the plasmids being modified by the DNA modifying enzymes. The plasmids are isolated and sequenced, preferably using nanopore sequencing. Using the UMIs, the sequences are then clustered. These clusters are used to construct accurate consensus sequences of the variant genes. A cluster thus refers to an enzyme variant identified with the methods of the present invention. Counting of the recombined plasmids generates an editing rate for the particular variant of the DNA modifying enzyme on the respective target site(s).

The method of the invention as laid out above can be likewise used for screening and sequencing of a plurality of target sites for DNA modifying enzymes. Accordingly, all embodiments and descriptions provided herein above apply likewise to a further aspect of the present invention, which provides a method for screening and sequencing of a plurality of target sites for DNA modifying enzymes, the method comprising the steps of:
providing a library of expression vectors comprising a first region encoding a DNA modifying enzyme, and a second region comprising variants of one or more two target sites of a DNA modifying enzyme;
introducing the library of expression vectors into host cells;
culturing the host cells and expressing the DNA modifying enzymes;
isolating plasmid DNA from the culture of host cells;
sequencing the first and the second region of the expression vectors; and
determining, based on the sequencing results, whether the DNA sequence of the second region on the expression vector has been altered by the DNA modifying enzyme.

In this second aspect of the present invention, the target sites in the second region of the vector can be different naturally occurring target site of different DNA modifying enzymes. In addition or alternatively, the target sites can be an artificially created target sites such as modified versions of a known target site. Such modified versions of a known target site preferably differ in one, two, three, four, five, six, seven, eight, nine or ten nucleotides from the known target site. A modified target site may alternatively differ from a known target site in about 2%, about 4%, about 6%, about 8%, about 10%, about 12%, about 14%, about 16%, about 18%, about 20%, about 25%, about 30%, about 35%, about 40%, about 45%, or in about 50% of the nucleotides.

The method preferably utilizes nanopore technology for sequencing full-length enzyme variants at fast turn-around times. By simultaneously capturing the target site with the enzyme sequence, the DNA editing rate for each enzyme variant can be quantified. Using unique molecular identifiers, the sequencing results can be clustered to a highly accurate consensus sequence.

The inventors were able to select DNA modifying enzymes from the enriched library which show a DNA editing rate, preferably a base and/or prime editing rate which is at least 10 %, 20% or 30 %, preferably at least 40 %, more preferably at least 50 %, most preferably at least 60 % higher, or 70 % or higher or 80 % higher or 90 % higher than the base editing rate of the original DNA modifying enzyme.

The invention further relates to a DNA modifying enzyme as described in the first aspect of the invention, which has been prepared and selected by the method of the second aspect of the invention.

The DNA modifying enzyme described in the first aspect of the invention may be comprised in in a pharmaceutical composition, which is suitable for use in certain medical therapies, such as gene therapy.

Accordingly, the invention further relates to use of the DNA modifying enzyme as described in the first aspect of the invention, or a pharmaceutical composition comprising the same in medicine.

The DNA modifying enzyme as described in the first aspect of the invention are also useful tools in research applications.

The DNA modifying enzyme as described in the first aspect of the invention are also useful tools in the generation of engineered plants in agriculture.

The applicability of the first and second aspects of the present invention is demonstrated in the following non-limiting examples using evolved Cas 12f-derived mini-ABEs.

### Examples

### Example 1: UMI fragment preparation

To generate a DNA fragment for ligation, a single stranded DNA oligonucleotide was ordered containing primer binding sites, restriction sites, and 50 random bases. There were two variants of this oligonucleotide depending on which pEVO plasmid (Buchholz and Stewart, 2001) it was intended to be used for. For screening of the evolved Cas12f-ABE variants, the "Cas12f UMI-tag" (SEQ ID NO: 3524) was used. To make these oligonucleotides double stranded, a 50 µl PCR was performed with 20 µM of the primers UMIprimer F and UMIprimer R (SEQ ID NOs: 3525 and 3526), 10 µM of the oligonucleotide, 10 µl of 5x MyTaq buffer and 1 µl MyTaq polymerase (Bioline). The PCR-cycler was set 94°C for 90 seconds, followed by 10 cycles of 15 seconds at 94°C, 15 seconds at 54°C and 15 seconds at 72°C. The resulting PCR product was digested with SbfI and XbaI for the Cas12f UMI-tag. The digest was then again cleaned up with the Isolate II PCR and Gel Kit (Bioline) and measured with a Qubit HS dsDNA Kit on a Qubit 2.0 (Thermo Fisher Scientific).

### Example 2: Enzyme variant barcoding

Evolved libraries of Cas12f-ABE were obtained by directed evolution of the fusion protein Un1Cas12f1-Tad8e (short name: Cas12f-ABE, SEQ ID NO: 1). The resulting enzyme variants contain randomly acquired mutations in comparison to their origin. The DNA editing enzyme gene variants were acquired from the pEVO plasmids used in the evolution by digesting with XbaI and BsrGI-HF for the evolved Cas12f-ABE library. The Cas12f-ABE library and the Cas12f-ABE controls were ligated in a ratio of 60 ng of Cas12f-ABE fragment, 4.8 ng UMI-tag and 100 ng of BsrgI-HF and SbfI digested pEVO-BE plasmid.

Ligated plasmids were desalted with MF-Millipore membrane filters (Merck) on distilled water for 30 minutes and transformed into XL-1 Blue E. coli (Agilent) via electroporation. The transformed bacteria were cultured in SOC medium for 30 minutes at 37°C. 2 µl of this culture was spread on agarose plates with 15 mg/ml chloramphenicol and incubated over night at 37°C. The number of colonies on the plates were counted to calculate the number of transformed bacteria present per µl of SOC culture.

To nominate the number of variants for the screen, an amount of the SOC culture equal to the desired number of variants was cultured overnight in 100 ml LB medium with 25 mg/ml chloramphenicol and a defined amount of L-arabinose. For the Cas12f-ABE screen, around 4,000 transformed bacteria per library and 100 transformed bacteria per control were cultured with 10 µg/ml L-arabinose. For each sequencing run, the different libraries and controls were cultured together and the plasmid DNA of these cultures was extracted with the GeneJet Plasmid Miniprep Kit (Thermo Fisher Scientific).

Barcoded plasmid extracts from the Cas12f-ABE library were digested with ScaI and BsrgI, The resulting fragments containing the evolved gene, the UMI-tag, and the target sites were isolated via agarose gel excision with the Isolate II PCR and Gel Kit (Bioline).

### Example 3: Nanopore sequencing and processing of screen libraries

DNA concentrations of the digested and isolated plasmid extracts (Example 2) were measured with a Qubit dsDNA HS Assay Kit on a Qubit 2.0 Fluorometer (Thermo Fisher Scientific). Nanopore sequencing library preparation for the Cas 12f-ABE library was performed using the protocol "Amplicons by Ligation (SQK-LSK112)". The Cas12f library was loaded onto a MinION FLO-MIN110 flow cell with r10.4 pores (Oxford Nanopore Technologies). Sequencing was performed for 72 hours. Each screen was performed on one flow cell.

Basecalling of the sequence data was performed on guppy version 6.0.1 with the super accuracy model for the Cas12f-ABE library (Oxford Nanopore Technologies). Processing of the sequence data was performed on a custom developed pipeline (available on https://github.com/ltschmitt/DEQSeq). Reads were first filtered with Filtlong v0.2.1 (https://github.com/rrwick/Filtlong) to be at least 2,900 bp long for the Cas12f-ABE screen. Further, Filtlong was also used to filter the reads for a minimum mean Phred quality value of 18 for the Cas12f-ABE screen. The sequences were then aligned with minimap2 (Li, 2021) to a reference sequence containing Un1Cas12f1-ABE8e (Cas12f-ABE screen, SEQ ID NO: 3540) and the UMI consisting of 50 random bases. To ensure coverage of the genes and the UMI, the aligned reads were filtered with samtools (Danecek et al., 2021) based on coordinates at the beginning of the enzyme gene and the end of the UMI.

UMIs were then extracted from the filtered alignment using the stackStringsFromBam function from the R package GenomicAlignments (Lawrence et al., 2013). UMIs were subsequently clustered with VSEARCH (Rognes et al., 2016) with a cluster_identity value of 0.7. Sequence reads from clusters with a minimum size of 50 reads were then transferred to separate files and aligned to the gene-UMI reference sequence. These separate read files and alignments were used to construct consensus sequences with racon (Vaser et al. 2017) followed by further polishing with medaka (https://github.com/nanoporetech/medaka), both with standard settings. The polishing process was run in parallel with GNU parallel (Tange 2023). Finally, gene sequences were extracted using the R package GenomicAlignments and translated to amino acids.

For the Cas12f-ABE screen, the base editing rate of the enzyme variants was determined by aligning the clustered reads to a reference sequence that contains the unedited target site (SEQ ID NO: 3541). Using the GenomicAlignments R package (Lawrence et al., 2013), the aligned stacks of four bases from position two to five counting from the TTTG PAM sequence on all three target sites were extracted. The base editing of Cas12f-ABE is expected on position three or four after the PAM sequence. Comparing this region can therefore provide information about potential editing of adjacent positions. Editing rates can then be determined by counting the correctly edited reads, non-edited reads and other editing outcome reads.

Results from each screen were combined and filtered for clusters with 50 reads or more. All further data processing and visualization was performed in R with the tidyverse (Wickham et al. 2019) and stringdist packages (van der Loo, 2014).

### Example 4: Extraction of enzyme variants

To validate the screens obtained as described in Example 3, enzyme variants were extracted from the screened libraries using PCR. Reverse primers specific for the UMI of the cluster of interest were designed ("Cas12f UMI-2 R (SEQ ID NO: 3542), "Cas12f UMI-3030 R (SEQ ID NO: 3543), "Cas12f UMI-3301 R (SEQ ID NO: 3544)) and used together with a universal forward primer (binding on the plasmid before the sequence encoding the enzyme in region 1 ("Evolution F (SEQ ID NO: 3545) for the Cas12f-ABE screen) to amplify enzyme genes. PCRs were performed with a high-fidelity polymerase (Herculase II Fusion DNA Polymerase, Agilent) and PCR products were digested with XbaI and BsrgI (Cas12f-ABE enzymes).

### Example 5: pEVO vectors for Un1Cas12f1-ABE8e

To generate a plasmid with target sites, oligonucleotides containing the target sites (SEQ ID NOs: 3547 and 3548) were annealed to form a DNA fragment that was cloned into a BglII digested pEVO backbone via Cold Fusion (System Biosciences). SgRNA scaffold fragments were synthesized (Twist Bioscience) and cloned into pEVO vector containing the target sites utilizing NsiI and NotI restriction enzymes. The pEVO plasmid containing the sgRNA scaffold and the target sites was then used as a template in a PCR reaction where three different sgRNA spacers were included in the reverse primers. In that way, generated sgRNA fragments were introduced to the pEVO vector in a stepwise manner: by cloning sgRNA1 using NotI and NsiI, by cloning sgRNA2 using NsiI, and by cloning sgRNA3 using XhoI and SalI. Un1Cas12f1 fragments were produced by Twist Bioscience and amplified via PCR, and the TadA gene was prepared via PCR by using the pABE8e-protein plasmid as template (Addgene plasmid # 161788). To create Cas 12f-ABE, both PCR products were used as template for an overlap-PCR. The Cas12f-ABE was then cloned into the pEVO-TS-sg1-sg2-sg3 vector using BsrGI and XbaI restriction enzymes. The expression of the Cas12f-ABEs was controlled by the araBAD L-arabinose inducible promoter system.

### Example 6: Evolution of Cas12f-ABE

A schematic illustration of the procedure is shown in Fig. 1. In a first step, the Cas12f-ABE library was generated using error-prone-PCR with a low-fidelity DNA polymerase (MyTaq, Bioline and Primers Evolution F and Evolution R (SEQ ID NOs: 3545 and 3546) and cloned into the vector using BsrGI and XbaI restriction enzymes. After transformation into XL-1 blue E. coli, expression of the enzymes was induced with 200 µg/ml L-arabinose. After expression of the base editors, the plasmids were isolated and digested with restriction enzymes (RE), which target the sgRNAs and their target sites. Because the base editing of the target site takes place on the restriction enzyme site, the digest of these enzymes will linearize the edited plasmid, while the non-edited plasmids will be cut into two fragments (Fig. 1). This process can be visualized and quantified using agarose gel electrophoresis (Fig. 2A). The next round of directed evolution was started with an error-prone-PCR on the enzymatically digested DNA. Only edited plasmids were replicated because primers (small arrows in Fig. 1) are placed in a way that only the edited DNA fragment is a valid template for PCR. The PCR product was then cloned into non-edited pEVO vectors, which signified the start a new evolution cycle. For increasing selection pressure, L-arabinose levels and thus induction of enzyme expression were lowered from 200 µg/ml to 10 µg/ml. Finally, to enrich the library for the DNA editing quantification sequencing screen, four cycles of evolution at 10 µg/ml L-arabinose were performed with a high-fidelity polymerase (Herculase II Fusion DNA Polymerase, Agilent) (Fig. 2C).

### Example 7: Base editing assay

A schematic illustration of the base editing assay is shown in Fig. 2A. Edited and non-edited pEVO plasmids (circles in top part) for base editing are digested with restriction enzymes (RE) that are specific for a sequence in the target site (quadratic box) and in the sgRNA array (grey). If the target site is edited (quadratic box with line), the RE-site is lost. Due to the different number of cuts, the number and sizes of the fragment changes. Digestion of non-edited plasmids results in two DNA fragments (smallest two fragments), digestion of edited plasmids results in one DNA fragment (biggest fragment). These fragments are visualized using agarose gel electrophoresis (schematic shown at the bottom). A mixture of edited and non-edited plasmids is shown on the right lane of the gel schematic. To the right of the gel, pictograms are used to indicate to which editing status the fragments belong to. A line with a box filled with a horizontal line indicates the edited single DNA fragment, the shorter lines with half a box indicate the two non-edited fragments that have been cut. For the assay, the same pEVO plasmid that was used in the examples before has been used. The Cas12f-ABE variants were cloned utilizing BsrGI and XbaI restriction enzymes. Expression of the variants was controlled by an L-arabinose inducible promoter system (araBAD). Base editing of the target site results in a loss of a restriction enzyme site. Therefore, the restriction digest will linearize the edited plasmid, whereas non-edited plasmids will result in two fragments, which can be detected with gel electrophoresis (Fig. 2A).

### Example 8: Modification of the E. Coli genome

Three different sgRNA were cloned into the pEVO plasmid, which target genomic DNA of *E. Coli.* Cas12f-ABE WT and the three variants were cloned into these vectors utilizing BsrGI and XbaI restriction enzymes. Modified vectors were subsequently transformed into *E. Coli* cells. After an overnight culture, the cells were spun down and resuspended in 200 µl ddH2O. This suspension was then heated to 95°C for 10 minutes and spun down again. The supernatant, which contained the gDNA, was used for a PCR reaction that produces a DNA fragment containing all three sgRNA target sites (Primers "E_Coli gDNA F" (SEQ ID NO: 3549) and "*E_Coli* gDNA R" (SEQ ID NO: 3550)). The PCR products were sequenced via Sanger sequencing and the editing rates were analyzed with EditR (Kluesner et al., 2018).

### Example 9: Screening of evolved Cas12f-ABEs

The method of the present invention was further assessed on a CRISPR-Cas based system, a schematic representation of CRISPR associated substrate linked directed evolution (CaSLiDE) method is shown in Fig. 3. Using this method, 46 directed evolution cycles were performed to generate a library of Un1Cas12f1-ABE8e (Cas12f-ABE) with improved editing efficiency compared to the original Cas12f-ABE (Fig. 2B). The evolved library was then further enriched for four cycles, by performing CaSLiDE with a high-fidelity PCR (Fig. 2C). This library was then screened with the method of the present invention, in which the original Un1Cas12f1-ABE8e (wild type, WT) was used as control. In total, the screen yielded more than 3.600 UMI-clusters with 50 or more reads, of which about 3.4 % clusters were identified as WT control (Fig. 4A). The determined median editing rates of the WT were 0.5% (target site 1), 16% (target site 2), and 6.2% (target site 3) (Fig. 4B). Of the 3.519 non-WT clusters, 58 had editing rates of more than 90% on all 3 target sites (Fig. 4C). Table II shows the base editing rates of the 3.519 non-WT clusters (SEQ ID Nos. 2-3520) compared to the original ("WT") enzyme of SEQ ID NO. 1. In Table II, TS means target site.

To validate these results, three variants with high activity on all three target sites (clusters 2 (SEQ ID NO: 471), 3030 (SEQ ID NO: 20), 3301 (SEQ ID NO: 3)); Fig. 4A) from the screened Cas12f-ABE library were extracted and evaluated using a base editing assay, schematically shown in Fig. 2A. In short, the assay is performed by digesting the CaSLiDE evolution plasmid with restriction enzymes that recognize the target sites (quadratic box, Fig 2A). Successfully edited plasmids (quadratic box with dash, Fig 2A) do not contain the restriction sites. Non-edited target sites are cut by the restriction enzyme (RE), while edited target sites are not cut due to the change in the restriction site. This results in different DNA fragments, which are visualized using agarose gel electrophoresis. The editing dependent difference in the fragments makes it possible to quantify the amount of edited and non-edited fragments. The assay showed that the variants have editing rates on all three target sites simultaneously of 91.1%, 60.8%, and 82.6%, while for the WT 0% was recorded (Figs. 5A and 5B). The variants were also tested on genomic DNA of E. coli. Three different sgRNAs were tested with the selected variants or the WT control. Editing results were analyzed by Sanger sequencing (Figs. 6A and 6B). The quantified base editing rates further validated the superior editing efficiencies of the variants identified using the method of the present invention over WT.

### Example 10: Comparing the evolved deaminase domain in the context of SpCas9

To illustrate the full scope and potential of CaSLiDE, we have compared the best deaminase domain with the current gold standard of the ABE8e editor (Richter, M. F. et al., 2020). By replacing the ABE8e deaminase domain with the deaminase domain of our best clone (3030, (SEQ ID NO: 20) in an SpCas9 background, we demonstrate that the new deaminase doubles the base editing efficiency (Fig. 8). Whereas the already clinically used ABE8e achieved an "A" to "G" conversion rate of 19,9%, the ABE3030 variant boosted base editing to 46% (Fig. 8). This example illustrates the importance of employing CaSLiDE for immediate practical relevance to therapeutic gene editing and highlights the improved amino acid sequence for base editing of the ABE3030 variant.

### Cited non-patent literature

Buchholz F., Hauber J. (2011). In vitro evolution and analysis of HIV-1 LTR-specific recombinases. Methods 53, 102-109.
Buchholz, F., Stewart, A.F. (2001). Alteration of Cre recombinase site specificity by substrate-linked protein evolution. Nat Biotechnol 19, 1047-1052.
Cong L., Ann Ran F., Cox D., Lin S. et al. (2013). Multiplex Genome Engineering Using CRISPR/Cas Systems. Science 339(6121), 819-823.
Danecek P., Bonfield J.K., Liddle J., Marshall J., Ohan V., Pollard M.O., et al. (2021). Twelve years of SAMtools and BCFtools. GigaScience 10(2):1-4.
Gaj T., Gersbach C.A., Barbas, C.F. (2013). ZFN, TALEN and CRISPR/Cas-based methods for genome engineering. Trends Biotechnol. 31(7), 397-405.
Hoersten J., Ruiz-Gómez G., Lansing F., Rojo-Romanos T., Schmitt L.T., Sonntag J., Pisabarro M.T., Buchholz F. (2021). Pairing of single mutations yields obligate Cre-type site-specific recombinases. Nucleic Acids Research; available from: https://doi.org/10.1093/nar/gkab1240.
Karst S.M., Ziels R.M., Kirkegaard R.H., Sørensen E.A., McDonald D., Zhu Q., Knight R., Albertsen M. (2021). High-accuracy long-read amplicon sequences using unique molecular identifiers with nanopore or PacBio sequencing. Nat Methods 18:165-169; available from: https://doi.org/10.1038% 2Fs41592-020-01041-y.
Kluesner M.G., Nedveck D.A., Lahr W.S., Garbe J.R., Abrahante J.E., Webber B.R., et al. (2018). EditR: A Method to Quantify Base Editing from Sanger Sequencing. CRISPR J. 1(3):239-50.
Lansing F., Mukhametzyanova L., Rojo-Romanos T., Iwasawa K., Kimura M., Paszkowski-Rogacz M., Karpinski J., Grass T., Sonntag J., Schneider P.M. (2022). Correction of a Factor VIII genomic inversion with designer-recombinases. Nature Communications 13; available from: https://doi.org/10.1038%2Fs41467-022-28080-7.
Lansing F., Paszkowski-Rogacz M., Schmitt L.T., Schneider P.M., Romanos T.R., Sonntag J., Buchholz F. (2019). A heterodimer of evolved designer-recombinases precisely excises a human genomic DNA locus. Nucleic Acids Research 48, 472-485.
Lawrence M., Huber W., Pagès H., Aboyoun P., Carlson M., Gentleman R., Morgan M.T., Carey V.J. (2013). Software for computing and annotating genominc ranges. PLoS Comput. Biol. 9, 1-10.
Li H. (2021). New strategies to improve minimap2 alignment accuracy. Bioinformatics 37(23):4572-4.
Rognes T., Flouri T., Nichols B., Quince C., Mahé, F. (2016). VSEARCH: a versatile open source tool for metagenomics. PeerJ 2016, 1-22.
Schindelin J., Arganda-Carreras I., Frise, E., Kaynig V. et al. (2012). Fiji: an open-source platform for biological-image analysis. Nature Methods, 9, 676-682.
Sheriff A., Guri I., Zebrowska P. et al. (2022). ABE8e adenine base editor precisely and efficiently corrects a recurrent COL7A1 nonsense mutation. Nature: Scientific Reports 12, 19643.
Stoddard B.L. (2006). Homing endonuclease structure and function. Quaterly Reviews of Biophysics, Cambridge University Press, 38(1).
Tange O. (2018). GNU Parallel 2018; available from: https://zenodo.org/record/1146014. Van der Loo M.P.J. (2014). The stingdist package for approximate string matching. The R Journal, 6, 111-122.
Vaser R., Sović I., Nagarajan N., Šikić, M. (2017). Fast and de novo genome assembly from long uncorrected reads. Genome Res. 27, 737-746.
Wang Y., Zhao Y., Bellas A., Wang Y, Au K.F. (2021). Nanopore sequencing technology, bioinformatics and applications. Nature Biotechnology 39, 1348-1365.
Wickham H., Averick M., Bryan J., et al. (2019). Welcome to the Tidyverse. J. Open Source Softw. 4, 1686.
Xin C., Yin J., Yuan S., Ou L., Liu M., Zhang W., Hu J. (2022). Comprehensive assessment of miniature CRISPR-Cas12f nucleases for gene disruption. Nature Communications, 13, Article no.: 5623.
Zurek P.J., Knyphausen P., Neufeld K., Pushpanath A., Hollfelder F. (2020). UMI-linked consensus sequencing enables phylogenetic analysis of directed evolution. Nature Communications 11; available from: https://doi.org/10.1038/s41467-020-19687-9.
Richter, M. F. et al., Phage-assisted evolution of an adenine base editor with improved Cas domain compatibility and activity. Nat Biotechnol. 2020 Jul;38(7):883-891; doi: 10.1038/s41587-020-0453-z.

## Claims

1. A DNA modifying enzyme comprising or consisting of a DNA editing protein and a Cas protein and a peptide linker,
wherein said DNA editing protein and said Cas protein are connected by said peptide linker; and
wherein said DNA modifying enzyme has been obtained by directed molecular evolution of an original enzyme; and
wherein said DNA modifying enzyme shows a base editing rate which is at least 10%, 20% or 30 %, preferably at least 40 %, more preferably at least 50 %, most preferably at least 60 % higher than the base editing rate of said wildtype enzyme.

2. The DNA modifying enzyme of claim 1, wherein said DNA modifying enzyme shows a editing rate which is at least 10%, 20% or 30 %, preferably at least 40 %, more preferably at least 50 %, most preferably at least 60 % higher than the editing rate of the original enzyme on at least one target site, preferably on two target sites, more preferably on three target sites of said DNA modifying enzyme.

3. The DNA modifying enzyme of claim 1 or 2, wherein said DNA editing protein is selected from the group consisting of deoxycytidine deamination-derived editors (CBEs) which facilitate C•G to T•A mutations, deoxycytidine deamination-derived editors (CGBEs) which facilitate C•G to G•C mutations, deoxyadenosine deamination-derived base editors (ABEs) which facilitate A•T to G•C mutations, and prime editing enzymes such as reverse transcriptases in combination with a prime editing guide (peg)RNA, which facilitate all types of DNA substitution, insertions and deletions on a target site.

4. The DNA modifying enzyme as claimed in any one of claims 1 to 3, wherein said Cas protein is selected from the group consisting of Cas9; Cas 12, such as Cas12a, Cas12b, Cas12c, Cas12d, Cas12f, Cas12i, Cas12j, Cas12g, Cas12h, TnpB, Casµ; and Cas13, such as Cas13a, Cas13b, Cas13c, Cas13d.

5. The DNA modifying enzyme as claimed in any one of claims 1 to 4, wherein said DNA editing protein is a base editing protein, preferably an ABE base editor protein and wherein said Cas protein is Cas12f.

6. The DNA modifying enzyme as claimed in any one of claims 1 to 5, wherein said peptide linker comprises or consists of 5 to 35 amino acids, preferably 32 amino acids.

7. The DNA modifying enzyme as claimed in any one of claims 1 to 6, wherein said ABE base editor protein is selected from the group consisting of proteins consisting of amino acids 1 to 166 of SEQ ID NOs. 2 to 3520 and wherein said ABE base editor protein comprises at least one single amino acid substitution, preferably wherein the single amino acid substitution is comprised in the catalytic region, preferably wherein the single amino acid substitution is at a position of a conserved amino acid in the catalytic region of said ABE base editor, when compared to the original ABE base editor protein, which has the amino acid sequence of amino acids 1 to 166 of SEQ ID NO. 1.

8. The DNA modifying enzyme as claimed in any one of claims 1 to 7, wherein said Cas12f protein is selected from the group consisting of amino acids 199 to 726 of SEQ ID NOs. 2 to 3520 and wherein said Cas12f nuclease comprises at least one single amino acid substitution, preferably wherein the single amino acid substitution is comprised in the catalytic region, preferably wherein the single amino acid substitution is at a position of a conserved amino acid in the catalytic region of said Cas12f protein, when compared to the amino acid sequence of the original Cas12f protein, which has the amino acid sequence of amino acids 199 to 726 of SEQ ID NO. 1.

9. The DNA modifying enzyme as claimed in any one of claims 1 to 7, wherein said peptide linker consists of amino acids 167 to 198 of SQ ID Nos. 2 to 3520 and comprises at least one single amino acid substitution, when compared to the amino acid sequence of the original linker, which has the amino acid sequence of amino acids 167 to 198 of SEQ ID NO. 1.

10. The DNA modifying enzyme as claimed in any one of claims 5 to 9, wherein said DNA modifying enzyme comprises single amino acid substitutions at least at one or at more positions selected from the group consisting of those listed in Table I:
| **Table I** | | |
|---|---|---|
| **Amino acid position no.** | **Amino acid in original enzyme (SEQ ID NO. 1)** | **Exchanged by** |
| 32 | V | I |
| 75 | I | T/A/V |
| 110 | R | H |
| 128 | R | C |
| 137 | A | V/M |
| 155 | F | L |
| 158 | Q | R |
| 166 | N | S/R |
| 182 | T | A/P |
| 186 | A | V/P/T |
| 189 | E | K/G/V |
| 196 | G | A/S/T |
| 249 | K | E |
| 255 | A | S/T |
| 295 | I | L/T |
| 296 | S | G |
| 328 | I | F |
| 361 | S | G/N |
| 363 | I | V |
| 406 | N | S |
| 409 | S | D/G |
| 441 | Q | R |
| 473 | K | R |
| 538 | F | L/S |
| 550 | H | R |
| 583 | T | S/A |
| 590 | E | K/R/G/V |
| 659 | E | K/V |
| 705 | N | D |

11. The DNA modifying enzyme as claimed in any one of claims 5 to 10, wherein the target sites of said DNA modifying enzyme are selected from the group consisting of target site 1 (SEQ ID NO: 3521), target site 2 (SEQ ID NO: 3522), target site 3 (SEQ ID NO: 3523).

12. The DNA modifying enzyme as claimed in any one of claims 5 to 11, wherein said DNA modifying enzyme shows an editing activity on each of target site 1 (SEQ ID NO: 3521), target site 2 (SEQ ID NO: 3522), target site 3 (SEQ ID NO: 3523) of more than 70 %, preferably of more than 80 %, more preferably of more than 90 %, most preferably of more than 95%.

13. The DNA modifying enzyme as claimed in any one of claims 5 to 12 which shows an editing activity on each of target site 1 (SEQ ID NO: 3521), target site 2 (SEQ ID NO: 3522), target site 3 (SEQ ID NO: 3523), wherein said DNA modifying enzyme is selected from the group consisting of SEQ ID NO. 2 to 3520.

14. The DNA modifying enzyme as claimed in any one of claims 5 to 12 which shows an editing activity on all three of target site 1 (SEQ ID NO: 3521), target site 2 (SEQ ID NO: 3522), target site 3 (SEQ ID NO: 3523) of equally to or more than 90 %, and wherein said DNA modifying enzyme is selected from the group consisting of SEQ ID NOs. 2, 3, 4, 5, 6, 7, 8, 9, 10, 11, 12, 13, 14, 16, 17, 18, 19, 20, 21, 22, 23, 24, 26, 27, 29, 30, 31, 32, 33, 35, 36, 37, 38, 39, 41, 42, 45, 47, 48, 49, 50, 51, 54, 56, 57, 60, 61, 63, 64, 67, 69, 70, 73, 75, 76, 77, 78, 79, 82, 83, 84, 87, 88, 89, 91, 92, 93, 94, 100, 104, 106, 107, 110, 111, 114, 116, 120, 124, 134, 135, 138, 143, 154, 165.

15. The DNA modifying enzyme as claimed in any one of claims 5 to 12 which is selected from the group consisting SEQ ID NOs. 2, 3, 4, 5, 6, 7, 8, 9, 10, 11, 12, 13, 14, 15, 16, 17, 18, 19, 20, 21, 22, 23, 24, 25, 26, 27, 28, 33, 101, 471,

16. A method of directed molecular evolution of a DNA modifying enzyme as claimed in any one of claims 1 to 15 or of any of the accompanying factors (or components) such as sgRNA scaffold, or pegRNA scaffold in the case of prime editing, said method comprising the steps of
a. Generation of a library of said DNA modifying enzyme,
b. Cloning said library in an expression vector containing the nuclei acid of at least one target site, preferably of 2 or 3 target sites of said DNA modifying enzyme,
c. Transformation of the expression vector of step b. into a host cell and expression of the library of said DNA modifying enzyme in said host cell;
d. Isolation of plasmids and digestion with the restriction enzymes, which target the nucleic acids of said target sides;
e. Selection of linearized plasmids containing a DNA edition in the nucleic acid of at least one target site, preferably 2 or 3 target sites,
f. Subjecting the linearized plasmids to a PCR or DNA shuffling and repeating steps b. to e. to enrich the library of DNA modifying enzymes.

17. The method of claim 16, wherein the expression said library of said DNA modifying enzyme in said host cell is performed in presence of L-arabinose.

18. The method of claim 16 or 17, wherein 2 to 100, such as 10, 20, 30, 40, 50, 60, 70, 80, 90 or 100 cycles of directed molecular evolution are performed.

19. The method as claimed in any one of claims 16 to 18, wherein said library of said DNA modifying enzyme is further enriched by high fidelity PCR.

20. The method according to 19, wherein said further enriched library of said DNA modifying enzyme is screened for the DNA editing rate, preferably the base and/or prime editing rate of the DNA modifying enzymes on at least one, preferably 2 or 3 target sites, said method comprising sequencing of the DNA modifying enzymes contained in said library and activity testing in a base editing assay in comparison to the original enzyme of said DNA modifying enzyme.

21. The method of claim 20, further comprising selecting of DNA modifying enzymes from the enriched library which show a DNA editing rate, preferably a base and/or prime editing rate which is at least 30 %, preferably at least 40 %, more preferably at least 50 %, most preferably at least 60 % higher than the base editing rate of said original DNA modifying enzyme.

22. The DNA modifying enzyme es claimed in any one of claims 1 to 15, which has been prepared and selected by the method as claimed in any one of claims 1 to 15.

23. A DNA modifying enzyme as claimed in any one of claims 1 to 15 and 22 for use in gene therapy methods.
